# EUROPÄISCHE PATENTANMELDUNG

(11) **EP 2 910 561 A1**
(43) Veröffentlichungstag der Anmeldung: **26.08.2015**
(21) Anmeldenummer: 14156512.7
(22) Anmeldetag: 25.02.2014
(51) Int. Cl.: C07H 15/04, A61K 31/7028, A61P 31/04, A61P 37/08, A61P 29/00

(54) **Sialinsäurederivate**

(71) Anmelder: Brossmer, Reinhard, 69120 Heidelberg (DE); Prescher, Horst, 4057 Basel (CH)
(72) Erfinder: Brossmer, Reinhard, 69120 Heidelberg (DE); Prescher, Horst, 4057 Basel (CH)
(74) Vertreter: Rippel, Hans Christoph

(57) **Zusammenfassung**

Sialinsäurederivate der Formel (I) 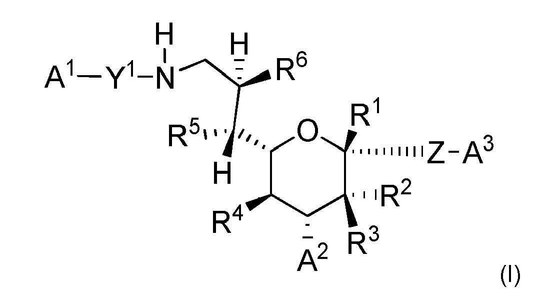 in der die Symbole die in der Beschreibung angegebenen Bedeutungen haben, eignen sich als Arzneimittel, insbesondere von Krankheiten, deren Verlauf durch Siclec-Liganden beeinflusst wird.

## Beschreibung

Die Erfindung betrifft Abkömmlinge der Sialinsäure, Verfahren zu deren Herstellung, deren Verwendung, insbesondere als pharmazeutische Wirkstoffe, sowie pharmazeutische Wirkstoffzusammensetzungen, die solche Verbindungen enthalten.

Sialinsäure ist der Oberbegriff für eine Familie von 9-Kohlenstoffatom-Zuckern, die sämtliche Derivate der Neuraminsäure (Neu) und der Keto-desoxy-nonuloson-Säure (KDN) darstellen. Typischerweise befinden sich diese an den exponierten nichtreduzierenden Enden von Oligosaccharidketten. Sialinsäuren spielen vielfältige Rollen in Säugetieren und im menschlichen Organismus (Schauer (2004) Zoology, 107, 49-64; Varki (2008) Trends in Mol. Med., 14, 8, 351- 360). Des Weiteren werden sie von vielen Pathogenen genutzt um z.B. eine effiziente Infektion zu erreichen oder um dem Immunsystem des Wirtes zu entkommen (Glycoconjugate J. 2006, vol. 23, issue 1-2, alle Artikel). Viele solcher Funktionen werden über Proteine reguliert, welche Sialinsäuren erkennen (Lehmann et al (2006) Cell- Mol. Life Sci. 63, 1331-1354).

Eine Untergruppe solcher Proteine sind die Siglecs. Sie sind Lektine vom Ig-Typ, die durch eine N-terminale V-Set Domäne gekennzeichnet sind, welche eine spezifische Erkennung von Sialinsäuren ermöglicht. Ein Überblick über die bis heute bekannt gewordenen Typen von Siglec-Proteinen und mit Siglec-Inhibitoren potentiell behandelbare Krankheiten findet sich in Trends in "Pharmacological Sciences 2009, 30 (5), 240-248" und "Current Medicinal Chemistry 2011, 18, 3537-3550" und den enthaltenen Referenzen.

CD22 (Siglec-2) ist stark auf B-Zellen exprimiert. Es ist bekannt dass B-Zell basierte Erkrankungen, insbesondere Lymphome und Autoimmunkrankheiten mittels CD22 Liganden behandelt werden können (Tedder et al (2005) Advances in Immunology 88, 1-50; Fiorina et al (2008) Diabetis 57, 3013-3024).

Weiter wurden bereits Antikörper und polymere Sialinsäuren als Liganden mit therapeutischer Eignung für Siglec-2 entwickelt (Courtney et al (2009) PNAS 106, 8, 2500-505; Collins et al (2006) Journal of Immunology 177, 2994-3003). Die Polymeren haben den Nachteil einer sehr hohen Molekulargewichtes, einer nicht definierbaren und nicht einheitlichen Größe und Zusammensetzung.

Es ist bekannt, dass bestimmte monomere Derivate der Sialinsäure mit Substitutionen an Position C-9 (WO 03/000709 und J. Exp. Med. 2002, 195, 9, 1207-1213), sowie mit Substitutionen an C-9, C-5 und C-2 (ChemMedChem 2012, 7, 134-143,) als Liganden für CD22 wirken und eine potentielle Eignung als Arzneimittel aufweisen. Weiter ist bekannt, dass Sialinsäurederivate mit Substitutionen an C-9 und C-4 und einer Methoxygruppe an C-2 (Angewandte Chemie Int. Ed., 2013, 53, 3616-3620) erhöhte Affinität zu Siglec-2 haben.

Dimere Derivate der Sialinsäure sind aus der WO 2013/190103 und WO 2013/097942 bekannt.

Obwohl die bekannten Derivate bereits hohe Affinitäten haben, besteht doch ein breiter Raum für Verbesserungen, insbesondere was Affinität und Selektivität betrifft. Weiter besteht Raum für Verbesserungen bezüglich pharmakologischer Verträglichkeit und Verabreichungsformen sowie der Stabilität in Plasma und Leber.

Aufgabe der Erfindung ist es, Verbindungen bereitzustellen, mit denen zumindest in Teilbereichen Vorteile in den genannten Bereichen erzielt werden.

Es wurde gefunden, dass sich bestimmte monomere Sialinsäure-Abkömmlinge mit einem an der 9-Position substituierten Stickstoff und weiteren Substituenten an der 4 und 2-Position in besonderer Weise als Siglec-2 (CD22) Liganden eignen.

Gegenstand der Erfindung ist daher ein Sialinsäurederivat der Formel (I), 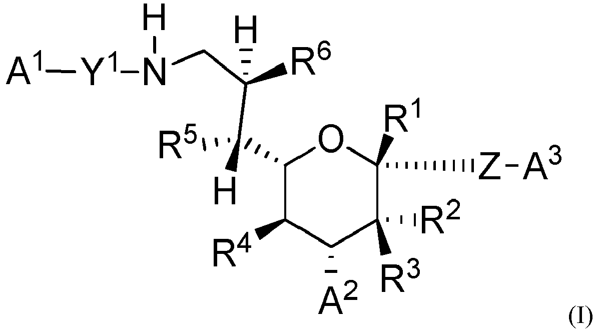 wobei die Symbole folgende Bedeutungen haben:
- A¹: ist eine Gruppe D¹-[Y²-D²-]ₘ-;
- D¹: ist ein mono- oder polycyclischer aromatischer, partiell ungesättigter oder gesättigter C₃-C₁₄-Kohlenwasserstoffrest oder ein mono- oder polycyclischer aromatischer, partiell ungesättigter oder gesättigter drei- bis zwölfgliedriger heterocyclischer Rest, wobei die genannten Reste unsubstituiert oder ein- oder mehrfach durch eine Gruppe X substituiert sind;
- D²: ist ein mono- oder polycyclischer aromatischer, partiell ungesättigter oder gesättigter C₃-C₁₄-Kohlenwasserstoffrest oder ein mono- oder polycyclischer aromatischer, partiell ungesättigter oder gesättigter drei- bis achtgliedriger heterocyclischer Rest, wobei die genannten Reste unsubstituiert oder ein- oder mehrfach durch eine Gruppe X substituiert sind;
- Y¹: ist ~C(O)-, ~S(O)₂-, ~NHC(O)-, ~(C₁-C₂-Alkyl)-, ~(C₁-C₂-Alkyl)-C(O)-, ~CH=CH-C(O)-, ~C≡C-C(O)-, ~(C₁-C₂-Alkyl)-S(O)₂-, ~OC(O)-, ~(C₁-C₂-Alkyl)-OC(O)- oder ~(C₁-C₂-Alkyl)-NHC(O)- wobei ~ die Bindung zur Gruppe A¹ bezeichnet;
- Y²: ist -O-, -C(O)-, -S(O)₂-, -CH₂- oder eine Bindung;
- A²: ist
a) eine Gruppe -OS(O)₂OL oder
b) eine Gruppe -N(R^{x})-W;
   W ist
   a) eine Gruppe ~SO₃L, ~SO₂CF₃ oder ~SO₂NR^{x}₂ oder
   b) eine Gruppe D³-Y³-;
- Y³: ist eine Bindung oder eine Gruppe ~O(CO)NHS(O)₂-, ~NHC(O)-, ~OC(O)-, ~CH₂OC(O)-, ~S(O)₂-, ~C(O)-, ~(C₁-C₂-Alkyl)-C(O)-, ~(C₁-C₂-Alkyl)-NHC(O)- oder ~(C₁-C₂-Alkyl)-S(O)₂-, wobei - die Bindung zur Gruppe D₃ bezeichnet;
- D³: ist
a) C₁-C₆-Alkyl, wobei gegebenenfalls eine oder mehrere nicht terminale CH₂-Gruppen durch O, N(R^{x}) und/oder C(O) ersetzt sind und wobei in den genannten Gruppen gegebenenfalls ein oder mehrere H-Atome durch eine Gruppe X ersetzt sind,
b) mono- oder polycyclischer aromatischer, partiell ungesättigter oder gesättigter C₃-C₁₄-Kohlenwasserstoffrest oder ein mono- oder polycyclischer aromatischer, partiell ungesättigter oder gesättigter drei- bis achtgliedriger heterocyclischer Rest, wobei die genannten Reste unsubstituiert oder ein- oder mehrfach durch eine Gruppe X substituiert sind;
- A³: ist
a) ein C₁-C₈-Alkyl, wobei gegebenenfalls
   a. eine oder mehrere nicht terminale -CH₂-Gruppen durch S, O, N(R^{x}) und/oder C(O) ersetzt sind oder
   b. eine -CH₂CH₂CH₂-Gruppe durch 1,2-Phenyldiyl, 1,3-Phenyldiyl oder 1,4-Phenyldiyl ersetzt ist und wobei in den genannten Gruppen gegebenenfalls ein oder mehrere H-Atome durch eine Gruppe X ersetzt sind.
b) ein mono- oder polycyclischer aromatischer, partiell ungesättigter oder gesättigter C₃-C₁₄-Kohlenwasserstoffrest oder ein mono- oder polycyclischer aromatischer, partiell ungesättigter oder gesättigter drei- bis achtgliedriger heterocyclischer Rest, wobei die genannten Reste unsubstituiert oder ein- oder mehrfach durch eine Gruppe X substituiert sind;
- X: ist gleich oder verschieden Halogen, Cyano, Nitro, Hydroxy, Mercapto, Amino, Carboxyl, Carboxymethyl, Hydroxylamino, Azido, B(OH₂), SO, SO₃M, OSO₃M, SO₂NH₂, SO₂CF₃, PO₃M, OPO₃M, Cyanomethyl, Alkyl, Halogenalkyl, Alkyloxy, Halogenalkyloxy, Alkylamino, Dialkylamino, Trialkylamino, Formyl, Alkylcarbonyl, Alkylsulfonyl, Alkylsulfoxyl, Alkylaminosulfonyl, Dialkylaminosulfonyl, Alkyloxycarbonyl, Alkylcarbonyloxy, Aminocarbonyl, Alkylaminocarbonyl, Alkylcarbonylamino, Dialkylaminocarbonyl, Oxo (=O), Thioxo (=S), C₁-C₈-Alkylimino (=N-C₁-C₈-Alkyl) oder C₁-C₈-Alkyloximino (=N-O-C₁-C₈-Alkyl), wobei die Alkyl-gruppen in diesen Resten 1 bis 6 Kohlenstoffatome enthalten;
- m: ist 0, 1 oder 2
- Z: ist ~O-, -S-, -ON=CH-, ~ON(R^{x})-, ~N(R^{x})- oder ~4-1H-(1,2,3)Triazol-1-yl- wobei ~ die Bindung zur Gruppe A³ bezeichnet;
- R¹: ist C(O)OM;
- R²: ist H, F, Cl, NR^{x}oder OR^{x};
- R³: ist H, F, Cl, NR^{x}oder OR^{x};
- R⁴: ist N(R^{x})C(O)CH₂OH oder N(R^{x})C(O)R^{x};
- R⁵, R⁶: sind gleich oder verschieden OH oder OR^{x};
- L: ist ein Kation;
- M: ist C₁-C₄-Alkyl oder ein Kation;
- R^{x}: ist gleich oder verschieden H, R^{y} oder R^{z};
- R^{y}: ist gleich oder verschieden C₁-C₄-Alkyl, Phenyl oder Benzyl und
- R^{z}: ist gleich oder verschieden -C(O)-C₁-C₄-Alkyl, -C(O)-Phenyl oder C(O)-CH₂-Phenyl.

Ebenfalls Gegenstand der Erfindung ist eine pharmazeutische Zubereitung, enthaltend mindestens ein Sialinsäurederivat der Formel (I) oder ein pharmakologisch verträgliches Salz oder Prodrug davon und einen pharmakologisch verträglichen Träger.

Weiterhin Gegenstand der Erfindung ist zudem ein Sialinsäurederivat der Formel (I) oder ein pharmakologisch verträgliches Salz oder Prodrug davon als Arzneimittel.

Beispielhaft werden Modifikationen einzelner Substituenten in pharmakologisch wirksamen Molekülen zu Prodrugformen beschrieben in Nature Drug Discovery Reviews, 2008, 7, 255-270 und in Hydrolysis in Drug and Prodrug Metabolism, Wiley-VCH, 2003, Bernard Testa und Joachim M. Mayer.

Gegenstand der Erfindung ist auch ein Sialinsäurederivat der Formel (I) oder ein pharmakologisch verträgliches Salz oder Prodrug davon zur Behandlung oder Prävention von Allergien, Autoimmunerkrankungen, chronischen Entzündungen, Querschnittslähmung, multipler Sklerose, Krebs, Viruserkrankungen, beispielsweise AIDS, bakteriellen Erkrankungen, beispielsweise Streptokokken, parasitären Erkrankungen, beispielsweise Chagas Krankheit, Krankheiten bei denen die Immunantwort im Rahmen der B-Zellenaktivierung gestört ist, wie Common Variable Immunodeficiency (CVID) und IgA-Defizienz, bei Krankheiten der blutbildenden Organe und des Blutes sowie bei Krebs, beispielsweise Lymphome und Myelome, sowie zur Regulation des Immunsystems, beispielsweise bei Impfungen.

Ein weiterer Gegenstand der Erfindung ist ein Sialinsäurederivat der Formel (I) oder ein pharmakologisch verträgliches Salz oder Prodrug davon zur Verwendung bei der Herstellung eines Arzneimittels zur Regulation des Immunsystems, beispielsweise bei Impfungen sowie zur Behandlung von Allergien, Autoimmunerkrankungen, chronischen Entzündungen, Querschnittslähmung, multipler Sklerose, Krebs, Viruserkrankungen, beispielsweise AIDS, bakteriellen Erkrankungen, beispielsweise Streptokokken, parasitären Erkrankungen, beispielsweise Chagas Krankheit, Krankheiten, bei denen die Immunantwort im Rahmen der B-Zellenaktivierung gestört ist, wie Common Variable Immunodeficiency (CVID) und IgA-Defizienz, bei Krankheiten der blutbildenden Organe und des Blutes sowie bei Krebs, beispielsweise Lymphome und Myelome.

Ebenfalls Gegenstand der Erfindung ist ein Verfahren zur Regulation des Immunsystems, beispielsweise bei Impfungen sowie zur Behandlung von Krankheiten, deren Verlauf oder Aktivität durch die Siglec-Liganden beeinflusst werden kann, insbesondere aus der Gruppe der Allergien, Autoimmunerkrankungen, chronischen Entzündungen, Querschnittslähmung, multipler Sklerose, Krebs, Viruserkrankungen, beispielsweise AIDS, Bakterielle Erkrankungen, beispielsweise Streptokokken, Parasitäre Erkrankungen, beispielsweise Chagas Krankheit, Krankheiten, bei denen die Immunantwort im Rahmen der B-Zellenaktivierung gestört ist, wie Common Variable Immunodeficiency (CVID) und IgA-Defizienz, bei Krankheiten der blutbildenden Organe und des Blutes sowie bei Krebs, beispielsweise Lymphome und Myelome, wobei man einer von der Krankheit betroffenen Person eine vorzugsweise therapeutisch wirksame Menge eines Sialinsäurederivats der Formel (I) oder eines pharmakologisch verträglichen Salzes oder Prodrugs davon verabreicht.

Die Sialinsäurederivate der Formel (I) weisen eine deutlich erhöhte Affinität im Vergleich zu bisher bekannten monovalenten CD22 Liganden auf. Sie beeinflussen *in vitro* bereits bei geringen Konzentrationen die Calciumausschüttung von B-Zellen.

Durch die Kombination von Substituenten C-9 und C-4 des Sialinsäuregerüstes mit größeren als dem bereits bekannten Methoxy-Subtituenten an C-2 wurde eine in unerwartet starkem Maße erhöhte die Affinität zu CD22 erzielt. Dies gilt insbesondere vor dem Hintergrund, dass für ein weiteres Mitglied der Siglec-Familie (MAG oder Siglec-4) die Kombination von Substituenten an Positionen C-9, C-4 und C-2 zu einer Erniedrigung der Affinität führte (Carbohydrate Research, 2010, 345, 1348-1359). Die Sialinsäurederivate der Formel (I) weisen eine hohe Aktivität zu CD22 auf und haben, im Gegensatz zu Polymeren, jeweils eine eindeutige und definierbare Struktur. Sie haben eine im Vergleich zu anderen bisher bekannten hochaffinen, dimeren CD22 Liganden ein deutlich verringertes Molekulargewicht. Sie enthalten zudem außer Sialinsäure keine weiteren Kohlenhydrate und können in einfacher Weise zu Prodrugs modifiziert werden. Die Herstellung der Verbindungen erfolgt ohne Einsatz von Zellkulturen oder Enzymen, wodurch eine Herstellung in großem Maßstab möglich ist.

Der Begriff "Sialinsäurederivat der Formel (I)" umfasst alle stereoisomeren Formen der Verbindung der Formel (I), insbesondere E/Z oder Cis/trans-Isomere bei substituierten Doppelbindungen oder Ringen und sowie Stereoisomere, die sich durch die Chiralitätszentren der Verbindungen der Formel (I) ergeben, insbesondere Enantiomere und Diastereoisomere in reiner Form oder in Form von Gemischen jeglicher Zusammensetzung, wobei die einzelnen Chiralitätszentren jeweils in der (S)- oder (R)-Form vorliegen.

Die Herstellung der einzelnen Stereoisomeren kann beispielsweise durch Aufkonzentrierung der Isomerengemische nach üblichen Methoden, wie Chromatographie oder Kristallisation, oder durch Verwendung von isomerenreinen Ausgangsstoffen erfolgen. Die Aufbesserung der Isomere kann auf Stufe der Edukte, Zwischenprodukte oder Endprodukte der Formel (I) erfolgen. Zu den erfindungsgemäß umfassten Isomeren zählen auch alle tautomeren Formen von Verbindungen (I) und alle mesomorphen Formen.
Weiterhin umfasst der Begriff "Sialinsäurederivat der Formel (I)" Solvate, beispielsweise Hydrate oder Addukte mit Alkoholen, sowie alle Kristallmodifikationen.

Gegenstand der Erfindung sind auch pharmakologisch wirksame Metaboliten der Verbindungen (I). Insbesondere umfasst der Begriff Metaboliten durch "in vivo" auftretende Enzyme wie Esterasen, Amidasen und andere Enzyme generierte Spaltprodukte.

Weiterhin umfasst der Begriff "Sialinsäurederivate der Formel (I)" pharmakologisch verträgliche Salze der Verbindungen (I), darunter auch interne (Zwitterionen).

Im Allgemeinen kommen die Salze derjenigen Kationen oder die Säureadditionssalze derjenigen Säuren in Betracht, deren Kationen, beziehungsweise Anionen, die pharmakologische Wirkung der Verbindungen (I) nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschten falls ein bis vier Wasserstoffatome durch R^{Y} ersetzt sein können, vorzugsweise Ammonium, Dimethylammonium, Diisopropylammonium, Tetramethylammonium, Triethylammonium, Tetrabutylammonium, 2-(2-Hydroxyeth-1-oxy)eth-1-yl-ammonium, Di-(2-hydroxyeth-1-yl)-ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)-sulfoxonium, in Betracht. Bevorzugt sind Na, Li, K, Ca, Mg und Ammonium (gegebenenfalls substituiert), besonders bevorzugt sind Na, Li und K, insbesondere bevorzugt ist Na.

Anionen von pharmakologisch verträglichen Säureadditionsalzen sind beispielsweise Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat, die Anionen von C₁-C₄-Alkansäuren, vorzugsweise Formiat, Acetat, Propionat und Butyrat, und anderen organischen Säuren, wie Pivalinsäure, Maleinsäure, Bernsteinsäure, Pimelinsäue, Fumarsäure, Apfelsäure, Sulfaminsäure, Phenylpropionsäure, Gluconsäure, Ascorbinsäure, Nicotinsäure, Zitronensäure und Adipinsäure.

Soweit nicht anders angegeben können Symbole, die mehrfach verwendet werden, unabhängig voneinander die gleichen oder verschiedenen Bedeutungen haben.

Die in der Formel (I) angegebenen Definitionen der Symbole bedeuten:
Halogen: Fluor, Chlor, Brom und lod;
Alkyl: gesättigte, geradkettige, verzweigte oder cyclische Kohlenwasserstoffreste mit beispielsweise 1 bis 8 Kohlenstoffatomen, wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1,1-Dimethylpropyl, 1-Ethylpropyl, Hexyl, 1,1-Dimethyl-butyl, 2,2-Dimethylbutyl, 3,3-Dimethylbutyl, 1,2-Dimethylbutyl, 1,3-Dimethylbutyl, 2,3-Di-methylbutyl, 1-Methylpentyl, 2-Methylpentyl, 3-Methylpentyl, 4-Methylpentyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Heptyl, Octyl, Cyclopropyl, Cyclobutyl, 1-Methylcyclopropyl, 2-Methylcyclopropyl, Cyclopentyl, 2,2-Dimethylcylopropyl, 2,3- Dimethylcylopropyl, Cyclohexyl und Cyclooctyl;
Halogenalkyl: geradkettige, verzweigte oder cyclische Alkylgruppen mit beispielsweise 1 bis 6 Kohlenstoffatomen (wie vorstehend genannt), wobei in diesen Gruppen Wasserstoffatome teilweise oder vollständig durch Halogenatome ersetzt sind: wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 1-Fluor-1-methyl-ethyl, 1-Fluorcyclopropyl, Heptafluorpropyl oder Nonafluorbutyl; Alkyloxy: Alkyloxygruppen mit gesättigtem, geradkettigem, verzweigtem oder cyclischen Alkylrest, wobei dieser aus der vorstehend genannten Gruppe der Alkyle ist und 1 bis 6 Kohlenstoffatome enthält;
Halogenalkyloxy: Halogenalkyloxygruppen mit geradkettigem, verzweigtem oder cyclischem Halogenalkylrest, wobei dieser aus der vorstehend genannten Gruppe der Halogenalkyle ist und 1 bis 6 Kohlenstoffatome enthält;
Alkylamino: Alkylaminogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Dialkylamino: Dialkylaminogruppen mit gesättigten, geradkettigen, verzweigten oder cyclischen Alkylresten, wobei diese gleich oder verschieden aus der vorstehend genannten Gruppe der Alkyle sind und 1 bis 4 Kohlenstoffatome enthalten;
Trialkylamino: Trialkylaminogruppen mit gesättigten, geradkettigen, verzweigten oder cyclischen Alkylresten, wobei diese gleich oder verschieden aus der vorstehend genannten Gruppe der Alkyle sind und 1 bis 4 Kohlenstoffatome enthalten;
Alkylcarbonyl: Alkylcarbonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylsulfonyl: Alkylsulfonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylsulfoxyl: Alkylsulfoxylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylaminosulfonyl: Alkylaminosulfonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Dialkylaminosulfonyl: Dialkylaminosulfonylgruppen mit gesättigten, geradkettigen, verzweigten oder cyclischen Alkylresten, wobei diese aus der vorstehend genannten Gruppe der Alkyle sind und 1 bis 4 Kohlenstoffatome enthalten;
Alkyloxycarbonyl: Alkyloxycarbonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylcarbonyloxy: Alkylcarbonyloxygruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylaminocarbonyl: Alkylaminocarbonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Dialkylaminocarbonyl: Dialkylaminocarbonylgruppen mit gesättigten, geradkettigen, verzweigten oder cyclischen Alkylresten, wobei diese aus der vorstehend genannten Gruppe der Alkyle sind und 1 bis 4 Kohlenstoffatome enthalten;
Alkylimino: Alkyliminogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkyloximino: Alkyloximinogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylcarbonylamino: Alkylcarbonylaminogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält.

Dem Fachmann ist bekannt, dass eine cyclische oder verzweigte Alkylgruppe mindestens drei Kohlenstoffatome aufweist. Erfindungsgemäß wird der Begriff "Alkyl" auch für Alkylengruppen (Alkandiylgruppen) gebraucht. Dies ist jeweils aus dem Zusammenhang ersichtlich.

Mono- oder polycyclischer, aromatischer, partiell ungesättiger oder gesättiger C₃-C₁₄-Kohlenwasserstoffrest bedeutet für D² beispielsweise:
a) C₆-C₁₄-Aryldiyl, insbesondere Phenylen-1,4-diyl, Phenylen-1,3-diyl, Phenylen-1,2-diyl, Naphthalin-1,2-diyl, Naphthalin-1,3-diyl, Naphthalin-1,4-diyl, Naphthalin-1,5-diyl, Naphthalin-1,6-diyl, Naphthalin-1,7-diyl, Naphthalin-1,8-diyl, Naphthalin-2,3-diyl, Naphthalin-2,6-diyl, Naphthalin-2,7-diyl, Biphenylen-1,2-diyl, Biphenylen-1,3-diyl, Biphenylen-1,4-diyl, Biphenylen-1,5-diyl, Biphenylen-1,6-diyl, Biphenylen-1,7-diyl, Biphenylen-1,8-diyl, Biphenylen-2,3-diyl, Biphenylen-2,6-diyl und Biphenylen-2,7-diyl;
b) C₃-C₈-Cycloalkyldiyl, insbesondere trans-Cyclopropan-1,2-diyl, Cyclopropan-1,1-diyl, trans-Cyclobutan-1,3-diyl, cis-Cyclobutan-1,3-diyl, trans-Cyclopentan-1,3-diyl, cis-Cyclohexan-1,4-diyl, trans-Cyclohexan-1,4-diyl, trans-Cycloheptan-1,4-diyl, trans-Cyclooctan-1,5-diyl und Cuban-1,4-diyl.

Monocyclischer aromatischer, partiell ungesättigter oder gesättigter drei- bis achtgliedriger heterocyclischer Rest bedeutet für D² beispielsweise:
a) nicht-aromatisches, gesättigtes oder teilweise ungesättigtes 5- oder 6-gliedriges Heterocyclodiyl, enthaltend ein bis drei Stickstoffatome und/oder ein Sauerstoff- oder Schwefelatom oder ein oder zwei Sauerstoff- und/oder Schwefelatome, insbesondere trans-Tetrahydrofuran-2,5-diyl, trans-Tetrahydrofuran-2,4-diyl, cis-Tetrahydrofuran-2,5-diyl, trans-Tetrahydrothien-2,5-diyl, trans-Tetrahydrothien-2,4-diyl, trans-Pyrrolidin-2,5-diyl, trans-Pyrrolidin-2,4-diyl, Isoxazolidin-2,4-diyl, Isoxazolidin-2,5-diyl, Isothiazolidin-2,4-diyl, Isothiazolidin-2,5-diyl, Pyrazolidin-1,3-diyl, trans-Oxazolidin-2,4-diyl, trans-Thiazolidin-2,5-diyl, Imidazolidin-1,3-diyl, trans-Imidazolidin-2,4-diyl, Pyrrolin-1,3-diyl, trans-Pyrrolin-2,4-diyl, trans-Pyrrolin-2,5-diyl, trans-Piperidin-2,5-diyl, Piperidin-1,4-diyl, trans-Dioxan-2,5-diyl, trans-Tetrahydropyran-2,5-diyl, trans-Hexa-hydropyridazin-3,6-diyl, trans-Hexahydropyridazin-1,4-diyl, trans-Hexahydropyrimidin-2,5-diyl, Hexahydropyrimidin-1,3-diyl, Hexahydropyrimidin-1,4-diyl, Piperazin-1,4-diyl, trans-Piperazin-2,5-diyl und Piperazin-1,3-diyl;
b) 5-gliedriges Heteroaryldiyl, enthaltend ein bis vier Stickstoffatome oder ein bis drei Stickstoffatome und/oder ein Schwefel- oder Sauerstoffatom, insbesondere Furan-2,4-diyl, Furan-2,5-diyl, Thiophen-2,4-diyl, Thiophen-2,5-diyl, Pyrrol-2,4-diyl, Pyrrol-2,5-diyl, Pyrazol-1,3-diyl, Oxazol-2,4-diyl, Oxazol-2,5-diyl, 1,3,4-Oxadiazol-2,5-diyl, 1,2,4-Oxadiazol-3,5-diyl, 1,2,4-Thiadiazol-3,5-diyl, 1,3,4-Thiadiazol-2,5-diyl, Isooxa-zol-3,5-diyl, Thiazol-2,4-diyl, Thiazol-2,5-diyl, Isothiazol-3,5-diyl, Imidazol-2,4-diyl, 2*H-*Tetrazol-2,5-diyl, 1*H*(1,2,4)-Triazol-2,5-diyl, 1*H*(1,2,3)-Triazol-1,4-diyl und 1*H*(1,2,3)-Triazol-1,5-diyl;
c) 6-gliedriges Heteroaryldiyl, enthaltend ein bis drei bzw. ein bis vier Stickstoffatome, insbesondere Pyridin-2,5-diyl, Pyridin-2,4-diyl, Pyridin-2,3-diyl, Pyridazin-3,6-diyl, Pyrimidin-2,5-diyl, Pyrimidin-2,6-diyl, Pyrazin-2,5-diyl und Tetrazin-3,5-diyl.

Polycyclischer aromatischer, partiell ungesättigter oder gesättigter heterocyclischer Rest bedeutet für D² beispielsweise:
1-Benzofuran-4,7-diyl, 1-Benzofuran-2,7-diyl, 2-Benzofuran-4,7-diyl, 2-Benzofuran-3,6-diyl, Chromen-5,8-diyl, Chromen-3,7-diyl, Xanthen-1,4-diyl, Xanthen-2,6-diyl, Indazol-4,7-diyl, Purin-2,8-diyl, 4H-Chinolizin-6,9-diyl, 3-Isochinolin-1,4-diyl, Phthalazin-1,4-diyl, 1,8-Naphthyridin-2,6-diyl, Chinoxalin-2,6-diyl, Chinazolin-5,8-diyl, Cinnolin-5,8-diyl, Pteridin-2,6-diyl, Indolizin-2,6-diyl, Indol-4,7-diyl, Indol-2,5-diyl, Indol-3,6-diyl, Isoindol-4,7-diyl, Isoindol-2,5-diyl, Carbozol-1,4-diyl, Acridin-1,4-diyl, Phenoxazin-1,4-diyl, Benzoxazol-4,7-diyl, Benzothiazol-4,7-diyl, Benzoimidazol-4,7-diyl, 1H-Benzotriazol-4,7-diyl und Benzothiophendiyl.

Mono- oder polycyclischer, aromatischer, partiell ungesättigter oder gesättigter C₃-C₁₄-Kohlenwasserstoffrest bedeutet für D¹, D³ und A³ beispielsweise:
a) C₆-C₁₄-Aryl, insbesondere Phenyl, 1-Naphthyl, 2-Naphtyl, 1-Biphenylen, 2-Biphenylen, 1-Pyrenyl, 1-Anthracenyl, 2-Anthracenyl, 9-Anthracenyl, 4-Indenyl, 2-Fluorenyl, 3-Fluorenyl, 9-Fluorenyl und 3-Phenanthrenyl;
b) C₃-C₁₄-Cy_{d}oalkenyl oder C₅-C₁₄-Cycloalkadienyl, insbesondere Cyclopropenyl, Cyclobutenyl, Cyclopentenyl, Cyclohexenyl, Cycloheptenyl, Cyclooctenyl, Cyclopentadien-1-yl, Cyclohexadien-1-yl und Cyclooctadien-1-yl;
c) C₃-C₈-Cycloalkyl, insbesondere Cyclopropyl, Cyclobutyl, Cyclopentyl, Cyclohexyl, Cycloheptyl, Adamantan-1-yl, Cuban-1-yl, Bicylo[4.4.0]decan-2-yl und Cyclooctyl.

Monocyclischer aromatischer, partiell ungesättigter oder gesättigter drei- bis achtgliedriger heterocyclischer Rest bedeutet für D¹, D³ und A³ beispielsweise:
a) nicht-aromatisches, gesättigtes oder teilweise ungesättigtes 4, 5, 6 oder 7-gliedriges Heterocyclyl, enthaltend ein bis vier Stickstoffatome und/oder ein Sauerstoff- oder Schwefelatom oder ein oder zwei Sauerstoff- und/oder Schwefelatome, insbesondere 1-Aza-2-yclobut-1-yl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 4,5-dihydro-1,3-oxazole-2-yl, 4,5-dihydro-1,3-oxazole-4-yl, 4,5-dihydro-1,3-oxazole-5-yl, 4,5-dihydro-1,3-thiazole-2-yl, 4,5-dihydro-1,3-thiazole-4-yl, 4,5-dihydro-1,3-thiazole-5-yl, 4,5-dihydro-4H-1,3-oxazin-2-yl, 4,5-dihydro-4H-1,3-thiazin-2-yl, 4,5,6,7-tetrahydro-1,3-oxazepin-2-yl, 4,5,6,7-tetrahydro-1,3-thiazepin-2-yl, 2-Thiazolidinyl, 4-Thiazolidinyl, 5-Thiazolidinyl, 2-Imidazolidinyl, 4-Imidazolidinyl, 5-Imidazolidinyl, 2-Pyrrolin-2-yl, 2-Pyrrolin-3-yl, 3-Pyrrolin-2-yl, 3-Pyrrolin-3-yl, 1-Piperidinyl, 2-Piperidinyl, 3-Piperidinyl, 4-Piperidinyl, 4-Morpholinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 5H-Tetrazol-5-yl, 1H-Tetrazol-5-yl, 2H-Tetrazol-5-yl 1-Piperazinyl und 2-Piperazinyl;
b) 5-gliedriges Heteroaryl, enthaltend ein bis vier Stickstoffatome oder ein bis drei Stickstoffatome und/oder ein Schwefel- oder Sauerstoffatom: insbesondere 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Isoxazolyl, 4-Isooxazolyl, 5-Isooxazolyl, 2-Thiazolyl, 4-Thiazolyl, 5-Thiazolyl, 2-Isothiazolyl, 4-Isothiazolyl, 5-Isothiazolyl, 2-Imidazolyl, 4-Imidazolyl, 1,2,5-Thiadiazol-3-yl, 1,2,4-Thiadiazol-3-yl, 1,2,4-Thiadiazol-5-yl, 1,2,5-Oxadiazol-3-yl, 1,2,4-Oxadiazol-3-yl, 1,2,4-Oxadiazol-5-yl, 1,2,3-Thiadiazol-4-yl, 1,2,3-Thiadiazol-5-yl, 1,2,3,4-Thiatriazol-5-yl, 1H-(1,2,3)Triazol-1-yl, 1 H-(1,2,3)Triazol-4-yl, 1H-(1,2,3)Triazol-5-yl, 1H-(1,3,4)Triazol-1-yl und 1H-(1,3,4)Triazol-2-yl;
c) 6-gliedriges Heteroaryl, enthaltend ein bis drei bzw. ein bis vier Stickstoffatome:
   insbesondere 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl und 2-Pyrazinyl;

Polycyclischer aromatischer, partiell ungesättigter oder gesättigter heterocyclischer Rest bedeutet für D¹, D³ und A³ beispielsweise:
1-Benzofuran-2-yl, 1-Benzofuran-3-yl, 1-Benzofuran-5-yl, 1-Benzofuran-6-yl, 1-Benzofuran-7-yl, 2-Benzofuran-1-yl, 2-Benzofuran-3-yl, 2-Benzofuran-4-yl, 2-Benzofuran-5-yl, 2-Benzofuran-6-yl, 2-Benzofuran-7-yl, 2H-Chromen-3-yl, 2H-Chromen-4-yl, 2H-Chromen-5-yl, 2H-Chromen-6-yl, 2H-Chromen-7-yl, 2H-Chromen-8-yl, Xanthen-1-yl, Xanthen-4-yl, Xanthen-9-yl, Indazol-1-yl, Indazol-3-yl, Indazol-4-yl, Indazol-5-yl, Indazol-6-yl, Indazol-7-yl, Purin-2-yl, Purin-6-yl, Purin-7-yl, Purin-8-yl, Chinolin-2-yl, Chinolin-3-yl, Chinolin-4-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, Isochinolin-8-yl, Phthalazin-1-yl, Phthalazin-3-yl, Phthalazin-5-yl, Phthalazin-6-yl, 1,8-Naphthyridin-2-yl, 1,8-Naphthyridin-3-yl, 1,8-Naphthyridin-4-yl, 1,8-Naphthyridin-6-yl, 1,8-Naphthyridin-7-yl, Chinoxalin-2-yl, Chinoxalin-5-yl, Chinoxalin-6-yl, Chinazolin-4-yl, Chinazolin-6-yl, Cinnolin-3-yl, Cinnolin-4-yl, Cinnolin-6-yl, Pteridin-2-yl, Pteridin-4-yl, Pteridin-6-yl, Pteridin-7-yl, Indolizin-1-yl, Indol-1-yl, Indol-2-yl, Indol-3-yl, Indol-5-yl, Indol-6-yl, Indol-7-yl, Indol-8-yl, Isoindol-1-yl, Isoindol-2-yl, Isoindol-4-yl, Isoindol-5-yl, Carbazol-9-yl, Acridin-9-yl, Phenoxazin-10-yl, 1-Benzo-thiophen-2-yl, 1-Benzothiophen-3-yl, 1-Benzothiophen-5-yl, 1-Benzothiophen-6-yl, 1-Benzothiophen-7-yl, 1-Benzothiophen-8-yl, Benzimidazol-1-yl, Benzimidazol-2-yl, Benzimidazol-5-yl, Benzimidazol-6-yl, Benzimidazol-7-yl, Benzimidazol-8-yl, 1H-Benzotrialzol-1-yl, 1H-Benzotrialzol-5-yl, 1H-Benzotrialzol-6-yl, 1H-Benzotrialzol-7-yl, 1 H-Benzotrialzol-8-yl, 4H-3,1-Benzoxazin-2-yl, 4H-2-Benzopyran-2-yl, 2H-Isochinolin-3-yl, Benzothiazol-2-yl, Benzothiazol-5-yl, Benzothiazol-6-yl, Benzothiazol-7-yl, Benzothiazol-8-yl, Benzoxazol-2-yl, Benzoxazol-5-yl, Benzoxazol-6-yl, Benzoxazol-7-yl oder Benzoxazol-8-yl.

Die genannten linearen oder cyclischen Kohlenwasserstoffreste und Heterocyclen können unsubstituiert oder substituiert sein, wobei die Substituenten vorzugsweise aus der Gruppe X gewählt sind. Bevorzugt sind, abhängig von der jeweiligen Ketten- oder Ringgröße, 1, 2, 3 oder 4 Substituenten; im Falle von Halogensubstituenten ist auch eine Substitution bis zur maximal möglichen Anzahl (Persubstitution) bevorzugt.

Vorteilhaft haben die Symbole in der Formel (I) folgende Bedeutungen:
- A¹: ist vorteilhaft eine Gruppe D¹-[Y²-D²-]ₘ-.
- D¹: ist vorteilhaft ein mono- oder polycyclischer aromatischer oder gesättigter C₃-C₁₄-Kohlenwasserstoffrest oder ein monocyclischer aromatischer, partiell ungesättigter oder gesättigter vier- bis sechsgliedriger heterocyclischer Rest, wobei die genannten Reste unsubstituiert oder ein- oder mehrfach durch eine Gruppe X substituiert sind.
- D²: ist vorteilhaft eine Gruppe Phenylen-1,4-diyl, Phenylen-1,3-diyl, Pyridin-2,5-diyl, Pyridazin-3,6-diyl, Pyrimidin-2,5-diyl, Pyrimidin-2,6-diyl, Pyrazin-2,5-diyl, trans-Cyclobutan-1,3-diyl, trans-Cyclopentan-1,3-diyl, trans-Cyclohexan-1,4-diyl, Cuban-1,4-diyl, Thiophen-2,5-diyl, Pyrrol-2,4-diyl, Pyrrol-2,5-diyl, Pyrazol-1,3-diyl, Oxazol-2,4-diyl, Oxazol-2,5-diyl, 1,3,4-Oxadiazol-2,5-diyl, 1,2,4-Oxadiazol-3,5-diyl, Isooxazol-3,5-diyl, Imidazol-2,4-diyl, 2*H*-Tetrazol-2,5-diyl, 1*H*(1,2,4)-Triazol-2,5-diyl, 1*H*(1,2,3)-Triazol-1,4-diyl und 1*H*(1,2,3)-Triazol-1,5-diyl wobei die genannten Reste unsubstituiert oder ein- oder mehrfach durch eine Gruppe X substituiert sind.
- Y¹: ist vorteilhaft ~C(O)-, ~S(O)₂-, -NHC(O)-, ~CH₂C(O)-, ~(CH₂-S(O)₂-, ~OC(O)-, ~CH₂-OC(O)- oder ~CH₂NHC(O)- wobei - die Bindung zur Gruppe A¹ bezeichnet.
- Y²: ist vorteilhaft -O-, -CH₂- oder eine Bindung.
- A²: ist vorteilhaft
a) eine Gruppe -OS(O)₂OL oder
b) eine Gruppe -N(R^{x})-W.
- W: ist vorteilhaft
a) eine Gruppe ~SO₃L, ~SO₂CF₃ oder ~SO₂NR^{x}₂ oder
b) eine Gruppe D³-Y³-.
- Y³: ist vorteilhaft eine Bindung oder eine Gruppe -NHC(O)-, ~OC(O)-, ~CH₂OC(O)-, ~S(O)₂-, ~C(O)-, ~CH₂-C(O)-, ~CH₂-NHC(O)- oder ~CH₂-S(O)₂-, wobei - die Bindung zur Gruppe D³ bezeichnet.
- D³: ist vorteilhaft
a) ein C₁-C₆-Alkyl, wobei gegebenenfalls eine oder mehrere nicht terminale CH₂-Gruppen durch O ersetzt sind und wobei in den genannten Gruppen gegebenenfalls ein oder mehrere H-Atome durch eine Gruppe X ersetzt sind,
b) ein monocyclischer oder polycyclischer aromatischer oder gesättigter C₃-C₁₄-Kohlenwasserstoffrest oder ein monocyclischer aromatischer, partiell ungesättigter oder gesättigter vier- bis sechsgliedriger heterocyclischer Rest, wobei die genannten Reste unsubstituiert oder ein- oder mehrfach durch eine Gruppe X substituiert sind.
- A³: ist vorteilhaft ein C₃-C₈-Alkyl, wobei gegebenenfalls
a. eine oder mehrere nicht terminale -CH₂-Gruppen durch S oder O ersetzt sind oder
b. eine -CH₂CH₂CH₂-Gruppe durch 1,2-Phenyldiyl, 1,3-Phenyldiyl oder 1,4-Phenyldiyl ersetzt ist und wobei in den genannten Gruppen gegebenenfalls ein oder mehrere H-Atome durch eine Gruppe X ersetzt sind;
   X ist vorteilhaft gleich oder verschieden Halogen, Hydroxy, Amino, Carboxyl, Carboxymethyl, SO₃M, OSO₃M, SO₂NH₂, SO₂CF₃, Alkyl, Halogenalkyl, Alkyloxy, Alkylamino, Dialkylamino, Trialkylamino, Alkylsulfonyl, Alkyloxycarbonyl, Alkylcarbonyloxy, Alkylaminocarbonyl, Alkylcarbonylamino oder Oxo (=O), wobei die Alkylgruppen in diesen Resten 1 bis 3 Kohlenstoffatome enthalten.
- m: ist 0 oder 1.
- Z: ist vorteilhaft ~O-, -S- oder ~4-1H-(1,2,3)Triazol-1-yl- wobei - die Bindung zur Gruppe A³ bezeichnet.
- R¹: ist vorteilhaft C(O)OM.
- R²: ist vorteilhaft H oder F.
- R³: ist vorteilhaft H, F, Cl oder OR^{x}.
- R⁴: ist vorteilhaft NHC(O)CH₂OH oder NHC(O)CH₃.
- R⁵, R⁶: sind vorteilhaft gleich oder verschieden OH oder OR^{z}.
- L: ist vorteilhaft ein Kation.
- M: ist vorteilhaft ein C₁-C₃-Alkyl oder ein Kation.
- R^{x}: ist vorteilhaft gleich oder verschieden H, R^{y} oder R^{z}.
- R^{y}: ist vorteilhaft gleich oder verschieden C₁-C₃-Alkyl, Phenyl oder Benzyl.
- R^{z}: ist vorteilhaft gleich oder verschieden -C(O)-C₁-C₃-Alkyl oder -C(O)-Phenyl.

Vorteilhaft bedeuten die in der Formel (I) angegebenen Definitionen der Symbole:
Halogen: Fluor, Chlor und Brom;
Alkyl: gesättigte, geradkettige, verzweigte oder cyclische Kohlenwasserstoffreste mit beispielsweise 1 bis 8 Kohlenstoffatomen, wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1,1-Dimethylpropyl, 1-Ethylpropyl, Hexyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 1-Methylpentyl, 2-Methylpentyl, 3-Methylpentyl, 4-Methylpentyl, 1-Ethylbutyl, 2-Ethylbutyl, 1,1,2-Trimethylpropyl, 1,2,2-Trimethylpropyl, 1-Ethyl-1-methyl-propyl, 1-Ethyl-2-methylpropyl, Heptyl, Octyl, Cyclopropyl, Cyclobutyl, 1-Methylcyclopropyl, 2-Methylcyclopropyl, Cyclopentyl, 2,2-Dimethylcylopropyl, 2,3- Dimethylcylopropyl, Cyclohexyl und Cyclooctyl;
Halogenalkyl: geradkettige, verzweigte oder cyclische Alkylgruppen mit beispielsweise 1 bis 3 Kohlenstoffatomen (wie vorstehend genannt), wobei in diesen Gruppen Wasserstoffatome teilweise oder vollständig durch Halogenatome ersetzt sind: wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 1-Fluor-1-methyl-ethyl, 1-Fluorcyclopropyl oder Heptafluorpropyl;
Alkyloxy: Alkyloxygruppen mit gesättigtem, geradkettigem, verzweigtem oder cyclischen Alkylrest, wobei dieser aus der vorstehend genannten Gruppe der Alkyle ist und 1 bis 3 Kohlenstoffatome enthält;
Alkylamino: Alkylaminogruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Dialkylamino: Dialkylaminogruppen mit gesättigten, geradkettigen, verzweigten oder cyclischen Alkylresten, wobei diese gleich oder verschieden aus der vorstehend genannten Gruppe der Alkyle sind und 1 bis 2 Kohlenstoffatome enthalten;
Trialkylamino: Trialkylaminogruppen mit gesättigten, geradkettigen, verzweigten oder cyclischen Alkylresten, wobei diese gleich oder verschieden aus der vorstehend genannten Gruppe der Alkyle sind und 1 bis 2 Kohlenstoffatome enthalten;
Alkylsulfonyl: Alkylsulfonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylaminosulfonyl: Alkylaminosulfonylgruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;
Alkyloxycarbonyl: Alkyloxycarbonylgruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;
Alkylcarbonyloxy: Alkylcarbonyloxygruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;
Alkylaminocarbonyl: Alkylaminocarbonylgruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;
Alkylcarbonylamino: Alkylcarbonylaminogruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;

Mono- oder polycyclischer, aromatischer oder gesättigter C₃-C₁₄-Kohlenwasserstoffrest bedeutet für D¹ und D³ beispielsweise:
a) C₆-C₁₄-Aryl, insbesondere Phenyl, Naphth-1-yl, Napht-2-yl, Biphen-4-yl, Biphen-2-yl, Anthracen-9-yl, Inden-4-yl, Fluoren-2-yl, Fluoren-3-yl, Fluoren-9-yl und Phenanthren-3-yl;
b) C₃-C₈-Cycloalkyl, insbesondere Cyclopropyl, Cyclobutyl, Cyclopentyl, Cyclohexyl, Cycloheptyl, Adamantan-1-yl, Cuban-1-yl, Bicylo[4.4.0]decan-2-yl und Cyclooctyl.

Monocyclischer aromatischer, partiell ungesättigter oder gesättigter drei- bis achtgliedriger heterocyclischer Rest bedeutet für D¹ und D³ beispielsweise:
a) nicht-aromatisches, gesättigtes oder teilweise ungesättigtes 4, 5 oder 6-gliedriges Heterocyclyl, enthaltend ein bis vier Stickstoffatome und/oder ein oder zwei Sauerstoffatome, insbesondere 1-Aza-cyclobut-1-yl, 2-Tetrahydrofuranyl, 3-Tetrahydrofuranyl, 2-Pyrrolidinyl, 3-Pyrrolidinyl, 3-Isoxazolidinyl, 4-Isoxazolidinyl, 5-Isoxazolidinyl, 3-Pyrazolidinyl, 4-Pyrazolidinyl, 5-Pyrazolidinyl, 2-Oxazolidinyl, 4-Oxazolidinyl, 5-Oxazolidinyl, 4,5-dihydro-1,3-oxazole-2-yl, 4,5-dihydro-1,3-oxazole-4-yl, 4,5-dihydro-1,3-oxazole-5-yl, 4,5-dihydro-4H-1,3-oxazin-2-yl, 2-Imidazolidinyl, 4-Imidazolidinyl, 5-Imidazolidinyl, 2-Pyrrolin-2-yl, 2-Pyrrolin-3-yl, 3-Pyrrolin-2-yl, 3-Pyrrolin-3-yl, 1-Piperidinyl, 2-Piperidinyl, 3-Piperidinyl, 4-Piperidinyl, 4-Morpholinyl, 1,3-Dioxan-5-yl, 2-Tetrahydropyranyl, 4-Tetrahydropyranyl, 3-Hexahydropyridazinyl, 4-Hexahydropyridazinyl, 2-Hexahydropyrimidinyl, 4-Hexahydropyrimidinyl, 5-Hexa-hydropyrimidinyl, 5H-Tetrazol-5-yl, 1H-Tetrazol-5-yl, 2H-Tetrazol-5-yl 1-Piperazinyl und 2-Piperazinyl;
b) 5-gliedriges Heteroaryl, enthaltend ein bis vier Stickstoffatome oder ein bis drei Stickstoffatome und/oder ein Schwefel- oder Sauerstoffatom: insbesondere 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Isoxazolyl, 4-Isooxazolyl, 5-Isooxazolyl, 2-Thiazolyl, 4-Thiazolyl, 5-Thiazolyl, 2-Isothiazolyl, 4-Isothiazolyl, 5-Isothiazolyl, 2-Imidazolyl, 4-Imidazolyl, 1,2,5-Thiadiazol-3-yl, 1,2,4-Thiadiazol-3-yl, 1,2,4-Thiadiazol-5-yl, 1,2,5-Oxadiazol-3-yl, 1,2,4-Oxadiazol-3-yl, 1,2,4-Oxadiazol-5-yl, 1,2,3-Thiadia-zol-4-yl, 1,2,3-Thiadiazol-5-yl, 1,2,3,4-Thiatriazol-5-yl, 1H-(1,2,3)Triazol-1-yl, 1 H-(1,2,3)Triazol-4-yl, 1 H-(1,2,3)Triazol-5-yl, 1H-(1,3,4)Triazol-1-yl und 1H-(1,3,4)Triazol-2-yl;
c) 6-gliedriges Heteroaryl, enthaltend ein bis drei Stickstoffatome: insbesondere 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl und 2-Pyrazinyl;

Die genannten linearen oder cyclischen Kohlenwasserstoffreste und Heterocyclen können unsubstituiert oder substituiert sein, wobei die Substituenten vorzugsweise aus der Gruppe X gewählt sind. Bevorzugt sind, abhängig von der jeweiligen Ketten- oder Ringgröße, 1, 2, 3 oder 4 Substituenten; im Falle von Halogensubstituenten ist auch eine Substitution bis zur maximal möglichen Anzahl (Persubstitution) bevorzugt.

Bevorzugt haben die Symbole in der Formel (I) folgende Bedeutungen:
- A¹: ist bevorzugt eine Gruppe D¹-[Y²-D²-]ₘ-.
- D¹: ist bevorzugt eine Gruppe Phenyl, Pyrimidin-5-yl, Napth-1-yl, Napth-2-yl, Thien-2-yl wobei die genannten Reste unsubstituiert oder ein- oder mehrfach durch eine Gruppe X substituiert sind.
- D²: ist bevorzugt eine Gruppe Phenylen-1,4-diyl, Pyridin-2,5-diyl, Pyrimidin-2,5-diyl oder Thiophen-2,5-diyl wobei die genannten Reste unsubstituiert oder ein- oder mehrfach durch eine Gruppe X substituiert sind;
- Y¹: ist bevorzugt ~C(O)- oder ~CH₂C(O)- wobei ~ die Bindung zur Gruppe A¹ bezeichnet.
- Y²: ist bevorzugt eine Bindung.
- A²: ist bevorzugt
a) eine Gruppe -OS(O)₂OL oder
b) eine Gruppe -NH-W.
- W: ist bevorzugt
a) eine Gruppe ~SO₃L oder
b) eine Gruppe D³-Y³-.
- Y³: ist bevorzugt eine Bindung oder eine Gruppe ~CH₂OC(O)-, -OC(O)-, ~S(O)₂-, ~C(O)-oder ~CH₂-C(O)- wobei - die Bindung zur Gruppe D³ bezeichnet.
- D³: ist bevorzugt eine Gruppe Methyl, Ethyl, Prop-2-yl, Pent-5-yl, Cyclopropyl, 1,1-Dimethylethyl, Phenyl, Thien-2-yl, Furan-2-yl, Imidazolidin-5-yl, Pyrazin-5-yl, Naphthalin-1-yl.
- A³: ist bevorzugt eine Gruppe Pentan-1-yl oder Hexan-1-yl.
- X: ist bevorzugt gleich oder verschieden Fluor, Chlor, Hydroxy, Carboxyl, Methyl, Trifluormethyl, Methoxy, Dimethylamino oder Oxo (=O);
- m: ist bevorzugt 0 oder 1.
- Z: ist bevorzugt ~O-.
- R¹: ist bevorzugt C(O)OM.
- R²: ist bevorzugt H.
- R³: ist bevorzugt H oder OH.
- R⁴: ist bevorzugt NHC(O)CH₃.
- R⁵, R⁶: sind bevorzugt gleich oder verschieden OH oder OC(O)CH₃.
- L: ist bevorzugt ein Kation.
- M: ist bevorzugt Methyl, Ethyl oder ein Kation.

Bevorzugt bedeuten die in der Formel (I) angegebenen Definitionen der Symbole:
Weiterhin bevorzugte Sialinsäurederivate der Formel (I) sind solche der Formeln (Ia) - (Ih), wobei die Symbole die in der Formel (I) angegebenen Bedeutungen und Bevorzugungen haben.

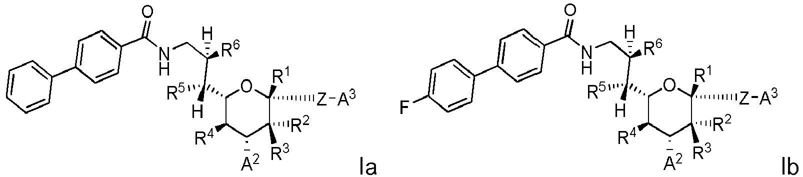

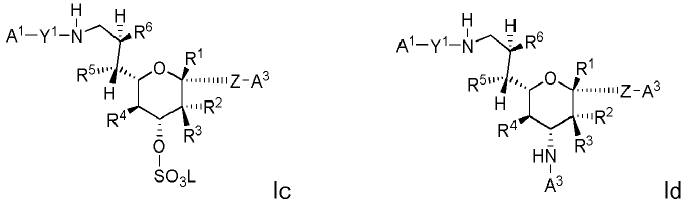

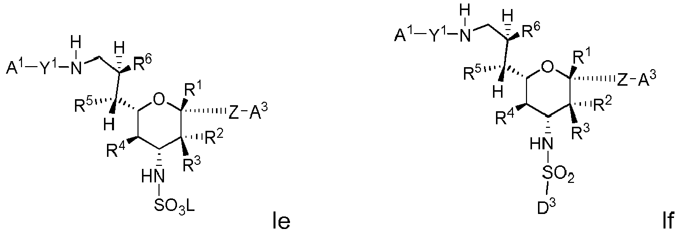

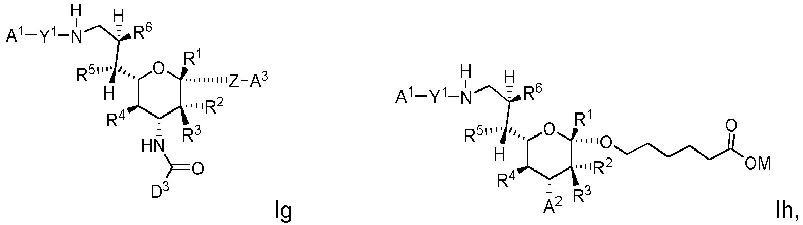

Besonders bevorzugt sind weiterhin Sialinsäurederivate der Formeln (Iaa)-(Iam), wobei die Symbole die in der Formel (I) angegebenen Bedeutungen haben:

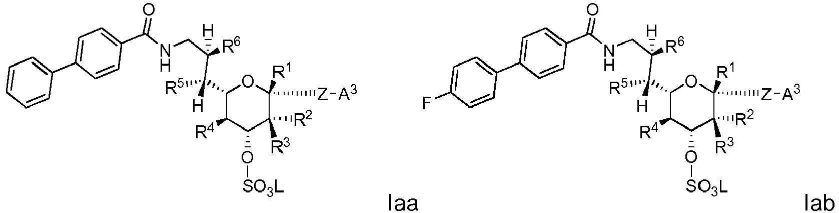

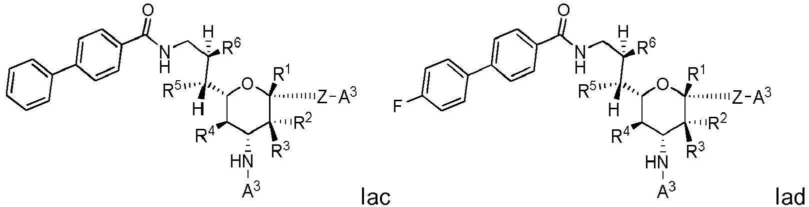

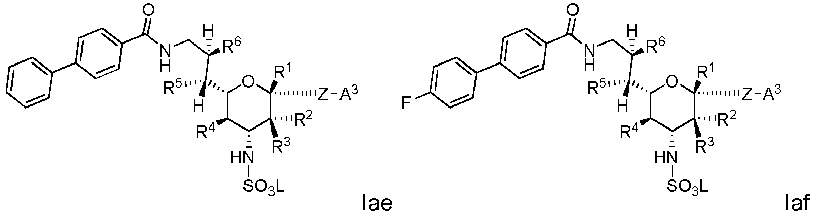

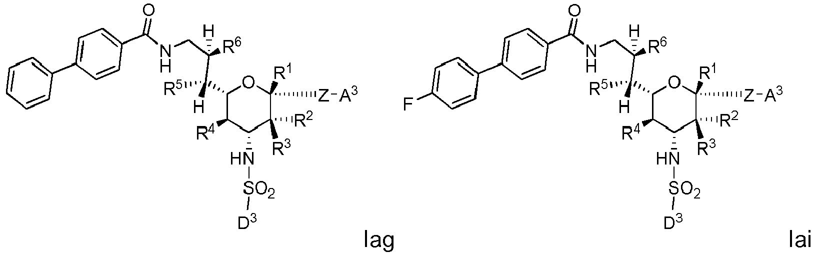

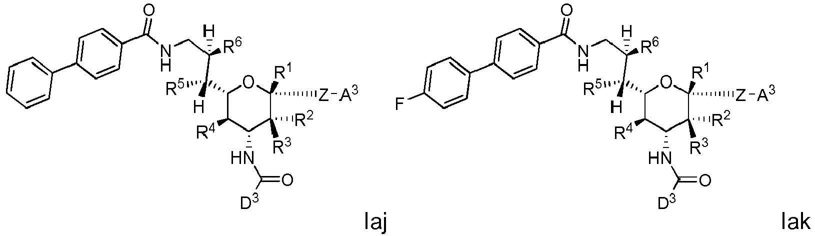

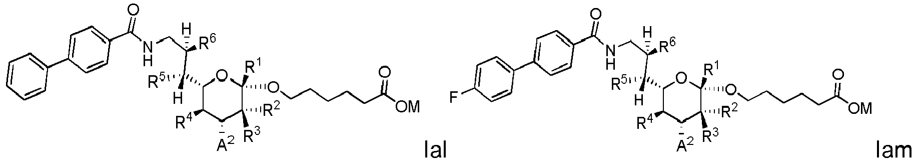

Insbesondere bevorzugt sind Sialinsäurederivate der Formeln (Iba)-(Ibj), wobei die Symbole die in der Formel (I) angegebenen Bedeutungen haben:

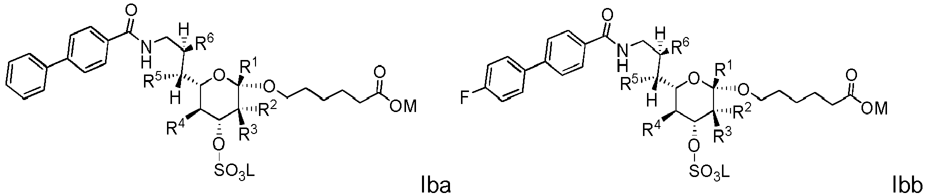

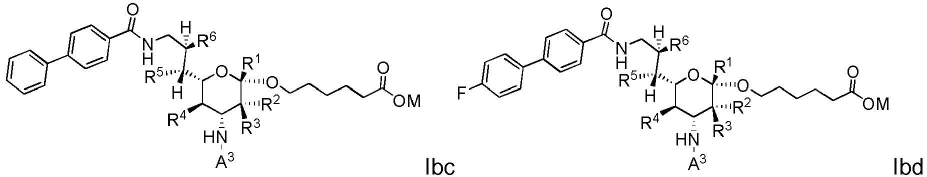

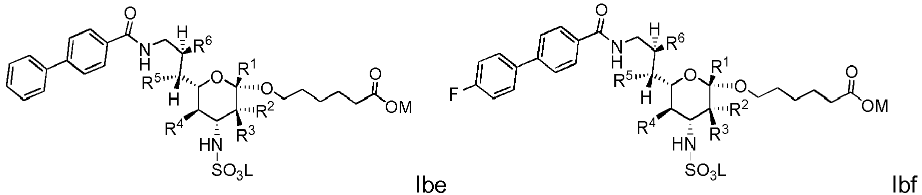

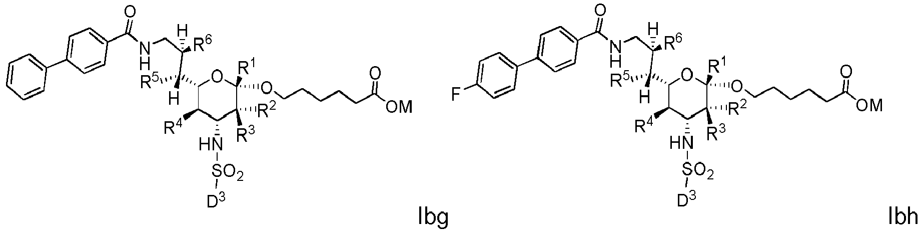

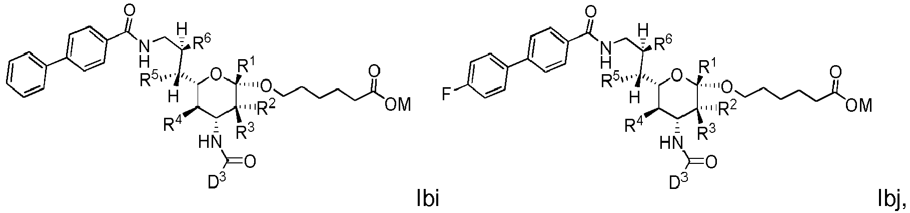

Stark bevorzugt sind Sialinsäurederivate der Formeln (Ica)-(Icj), wobei die Symbole die in der Formel (I) angegebenen Bedeutungen haben:

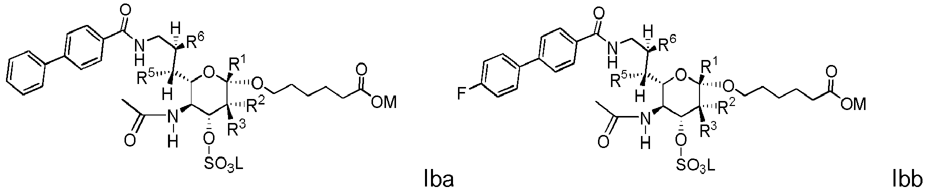

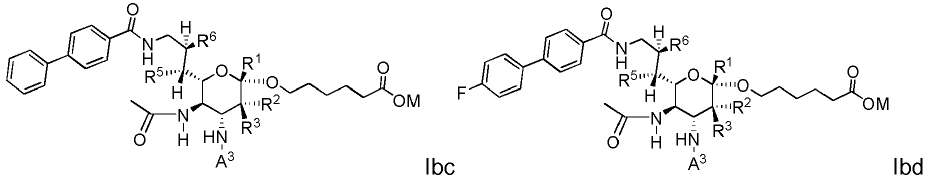

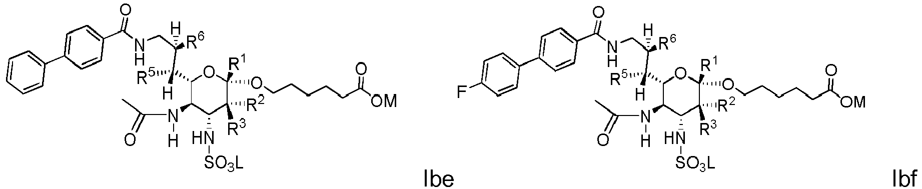

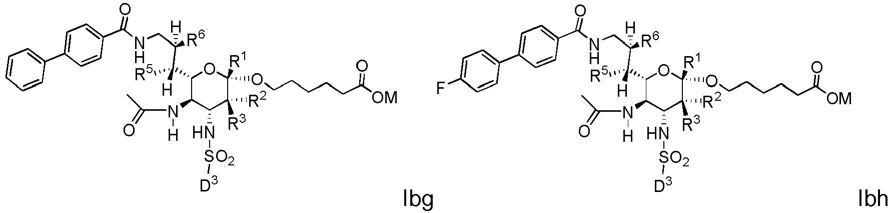

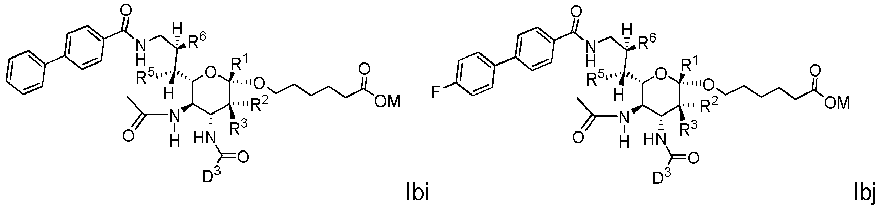

Die erfindungsgemäßen Sialinsäurederivate (I) sind unter Anwendung im Prinzip bekannter Syntheseverfahren erhältlich. Bevorzugt werden die Verbindungen nach den nachfolgend näher erläuterten erfindungsgemäßen Herstellungsverfahren, insbesondere den Syntheseschemata I - VII, erhalten:
Die Herstellung von Intermediaten zur Herstellung von Sialinsäurederivaten der Formel (I) kann beispielsweise gemäß den Syntheseschemata I bis IV erfolgen.

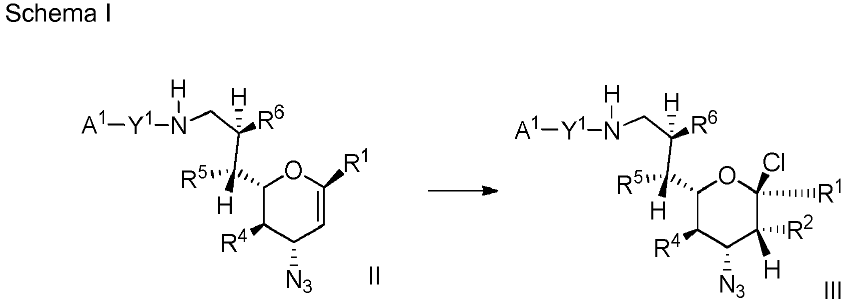

Verbindungen der Formel (III) können beispielsweise durch Addition von Chlorwasserstoff an die Doppelbindung in Verbindung (II) hergestellt werden. Beispielhaft wurde Verbindung 5 hergestellt.

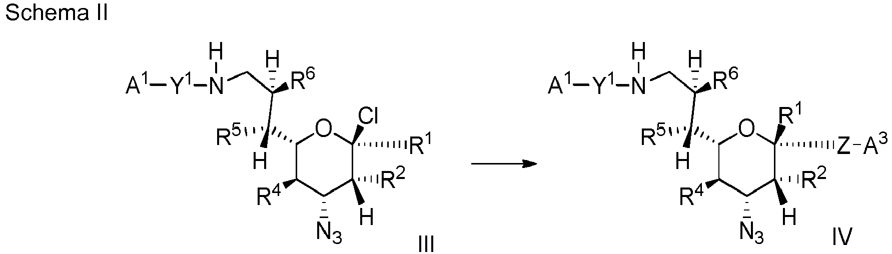

Verbindungen der Formel (IV) können beispielsweise durch Austausch des Chloratoms in Verbindung (III) durch die Gruppe Z¹-A³ hergestellt werden. Beispielhaft wurde Verbindung 13 hergestellt.

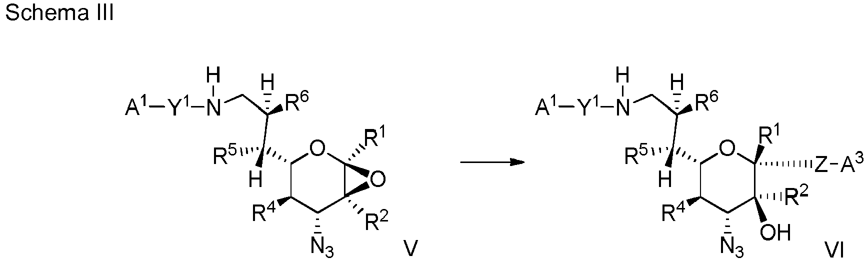

Verbindungen der Formel (VI) können beispielsweise durch Öffnen des Epoxides in Verbindung (V) durch die Gruppe Z¹-A³ hergestellt werden. Beispielhaft wurde Verbindung 8 hergestellt.

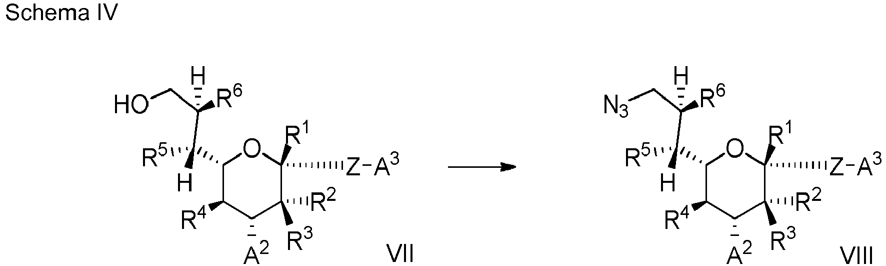

Verbindungen der Formel (VIII) können beispielsweise durch Austausch der Hydroxylgruppe in Verbindung (VII) durch ein Azid hergestellt werden. Beispielhaft wurde Verbindung 34 hergestellt.

Die Herstellung von Sialinsäurederivaten der Formel (I) kann beispielsweise gemäß den Syntheseschemata V bis VII erfolgen.

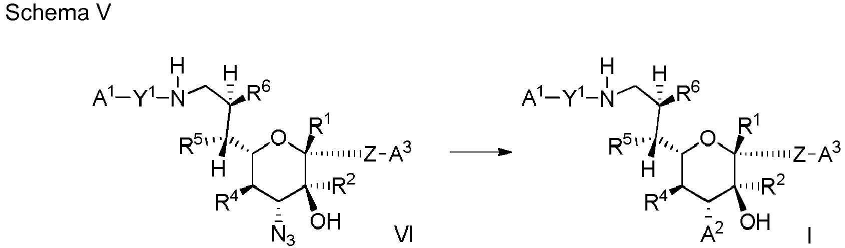

Verbindungen der Sialinsäurederivate der Formel (I) können beispielsweise durch Reduktion des Azids in Verbindungen der Formel (VI) und anschliessenden Umsatz des entstehenden Amines mit einer Carbonsäure oder einem Sulfonylchlorid hergestellt werden. Eventuell vorhandene Schutzgruppen können durch bekannte Verfahren entfernt werden. Beispielhaft wurden Verbindungen 10, 11, 38, 39, 40, 41, 42,43, 44, 45, 46, 47, 48, 49, 50, 51, 52 und 65 hergestellt.

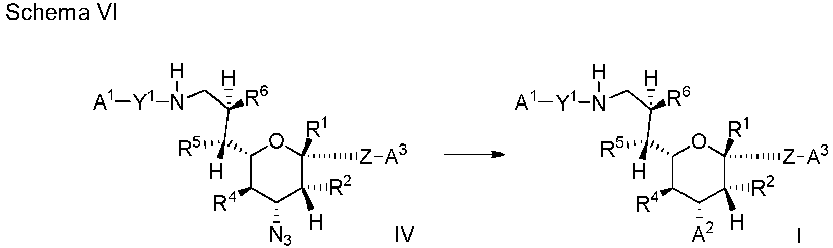

Verbindungen der Sialinsäurederivate der Formel (I) können beispielsweise durch Reduktion des Azids in Verbindungen der Formel (IV) und anschließenden Umsatz des entstehenden Amins mit einer aktivierten Carbonsäure, einem Sulfonylchlorid oder einem Sulfatierungsmittel hergestellt werden. Eventuell vorhandene Schutzgruppen können durch allgemein bekannte Verfahren entfernt werden. Beispielhaft wurden Verbindungen 15, 16, 17, 18, 53, 54, 55, 56, 57, 58, 59, 60, 61, 62, 63 und 64 hergestellt.

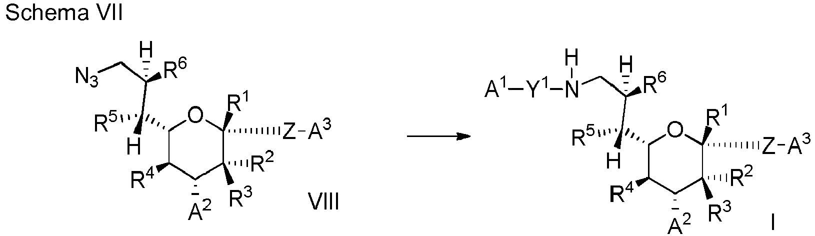

Verbindungen der Formel (I) können beispielsweise durch Reduktion des Azides in Verbindungen der Formel (VIII) und anschließendem Umsatz des entstehenden Amins mit einer aktivierten Carbonsäure, einem Sulfonylchlorid oder einem Sulfatierungsmittel hergestellt werden. Eventuell vorhandene Schutzgruppen können durch allgemein bekannte Verfahren entfernt werden. Beispielhaft wurde Verbindung 36 hergestellt.

Die Sialinsäurederivate der Formel (I) eignen sich als pharmakologisch wirksame Verbindungen und Wirkstoffe für Arzneimittelzubereitungen. Sie wirken als Siglec-Liganden, insbesondere als Siglec-2 (CD22) Liganden, zur Regulation des Immunsystems, insbesondere als Hilfsstoff bei Impfungen sowie zur Behandlung von Krankheiten, deren Verlauf oder Aktivität durch die Siglec-Liganden beeinflusst werden kann insbesondere Allergien, Autoimmunerkrankungen, chronischen Entzündungen, Querschnittslähmung, multipler Sklerose, Krebs, Viruserkrankungen, beispielsweise AIDS, sowie bei bakterielle Erkrankungen, beispielsweise Streptokokken, parasitäre Erkrankungen, beispielsweise Chagas Krankheit, Krankheiten, bei denen die Immunantwort im Rahmen der B-Zellenaktivierung gestört ist, wie Common Variable Immunodeficiency (CVID) und IgA-Defizienz, bei Krankheiten der blutbildenden Organe und des Blutes sowie bei Krebs, beispielsweise Lymphome und Myelome. Bevorzugte Indikationen sind Allergien, Autoimmunerkrankungen und CVID.

Behandlung bedeutet im Sinne der Erfindung eine therapeutische Behandlung, sowohl zur Heilung als auch zur Linderung von Symptomen, wie auch eine vorbeugende Behandlung.

Die Sialinsäurederivate können in Kombination mit anderen pharmakologisch wirksamen Substanzen, insbesondere solchen, welche die Wirksamkeit der erfindungsgemäßen Verbindungen verstärken, eingesetzt werden.

Gegenstand der Erfindung ist auch ein Verfahren zur Behandlung einer Siglec vermittelten Krankheit, insbesondere aus der Gruppe der Allergien, Autoimmunerkrankungen, chronischen Entzündungen, Querschnittslähmung, multipler Sklerose, Krebs, Viruserkrankungen, beispielsweise AIDS, Krankheiten, bei denen die Immunantwort im Rahmen der B-Zellenaktivierung gestört ist, wie Common Variable Immunodeficiency (CVID) und IgA-Defizienz, wobei man einer von der Krankheit betroffenen Person eine vorzugsweise therapeutisch wirksame Menge eines Sialinsäurederivats der Formel (I) verabreicht.

Weiterhin Gegenstand der Erfindung ist ein Sialinsäurederivat der Formel (I) oder ein pharmakologisch verträgliches Salz davon als Arzneimittel, insbesondere zur Behandlung von Siglec-vermittelten Krankheiten, wie den oben beschriebenen.

Gegenstand der Erfindung ist zudem ein Sialinsäurederivat der Formel (I) oder ein pharmakologisch verträgliches Salz davon zur Verwendung in einem Verfahren zur Behandlung von Siglec-vermittelten Erkrankungen, insbesondere den oben beschriebenen.

Ein weiterer Gegenstand der Erfindung ist ein Sialinsäurederivat der Formel (I) zur Verwendung zur Herstellung eines Arzneimittels, zur Behandlung von Siglec vermittelten Krankheiten, insbesondere den oben beschriebenen.

Ebenfalls Gegenstand der Erfindung ist eine pharmazeutische Zubereitung (=Arzneimittel), enthaltend mindestens ein Sialinsäurederivat der Formel (I) oder ein pharmakologisch verträgliches Salz davon und einen pharmakologisch verträglichen Träger.

Die zur Erzielung einer entsprechenden Wirkung bei der Behandlung oder Prophylaxe erforderliche Dosierung hängt üblicherweise von der zu verabreichenden Verbindung, vom Patienten, von der Art und Schwere der Krankheit oder des Zustandes und der Art und Häufigkeit der Verabreichung ab und liegt im Ermessen des zu behandelnden Arztes. Zweckmäßigerweise kann die Dosierung bei intravenöser Gabe im Bereich von 0,1 bis 1000 mg, vorzugsweise 0,5 bis 500 mg, und bei oraler Gabe im Bereich von 1 bis 1000 mg vorzugsweise 10 bis 500 mg, jeweils ein- oder mehrmals täglich, liegen. Hierzu lassen sich die erfindungsgemäßen Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Michzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Lösungen, Suspensionen oder Zäpfchen bearbeiten.

Das Verabreichen der erfindungsgemäßen Sialinsäurederivate (I) kann durch jede konventionelle Methode erfolgen, einschließlich oraler und parenteraler, z.B. durch intravenöse, subkutane oder intramuskuläre Injektionen.

Die Sialinsäurederivate (I) können auch für andere als die genannten Zwecke eingesetzt werden, beispielsweise als Diagnostika, z.B. in Verfahren zur Bestimmung der Aktivität von Siglec-Liganden, als biochemische Sonden oder als Zwischenprodukte zur Herstellung weiterer, insbesondere pharmakologisch aktiven Verbindungen.

Die Erfindung wird durch die Beispiele näher erläutert ohne sie dadurch zu beschränken.

### Beispiele

### A. Synthesebeispiele werden in Schemata 1 bis 18 dargestellt.

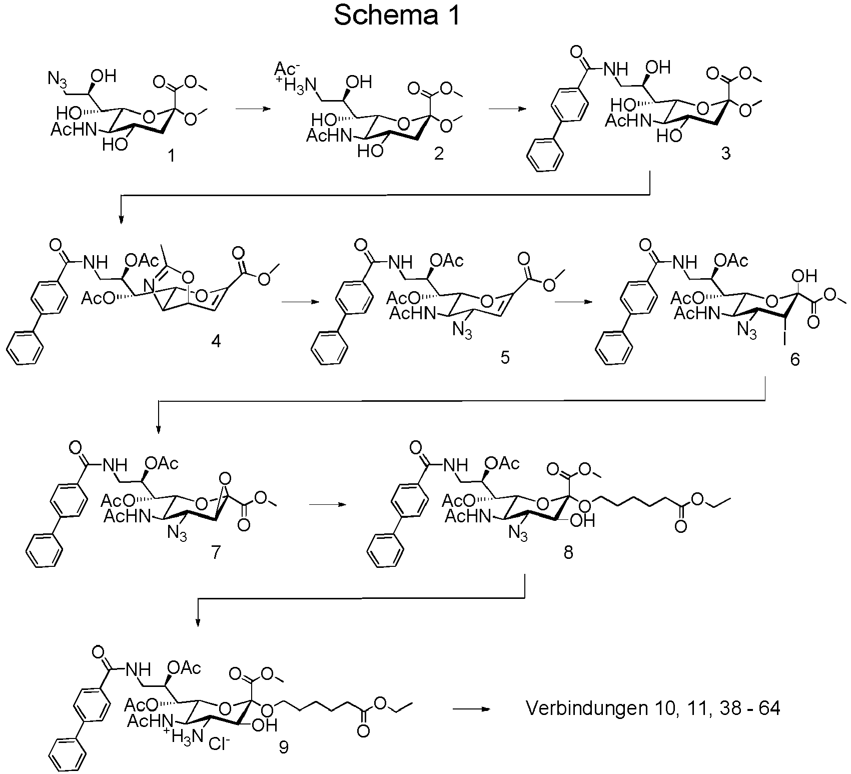

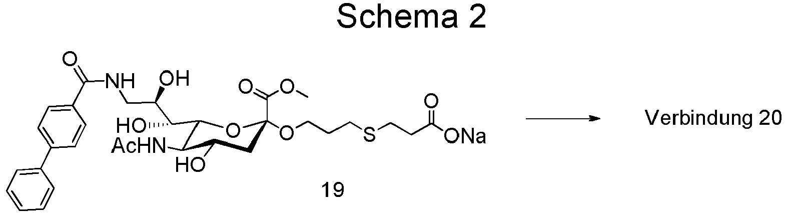

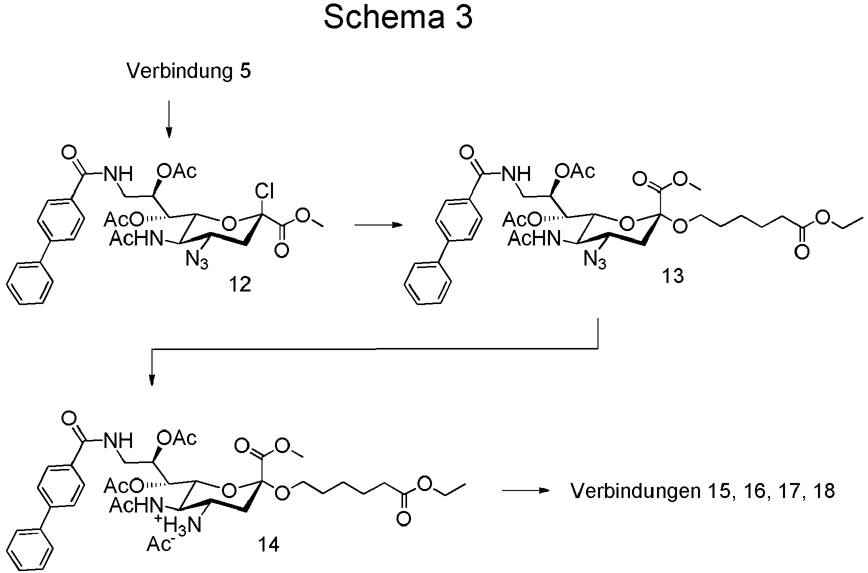

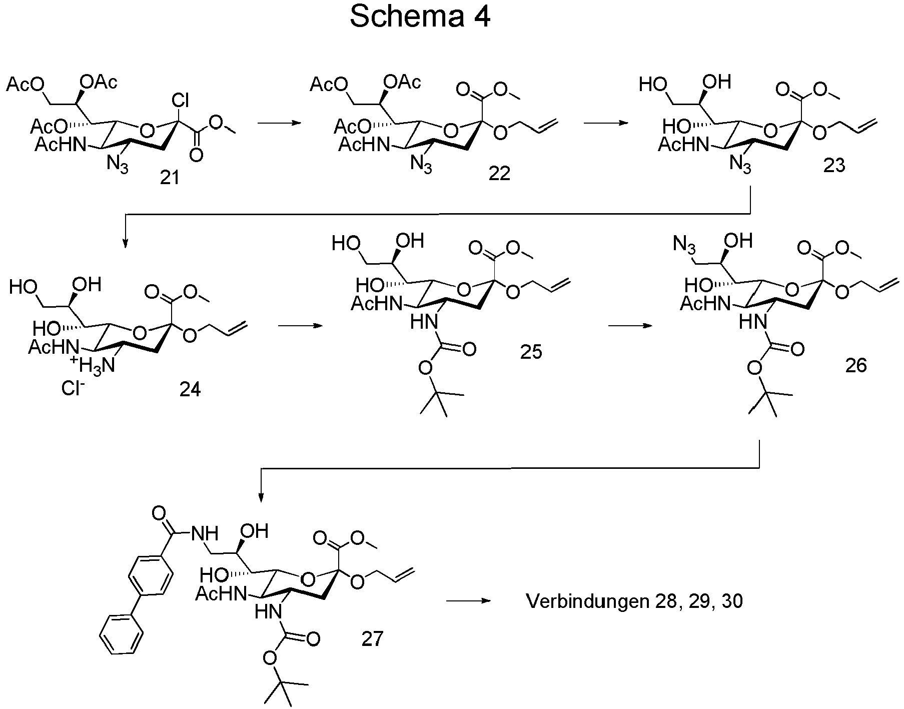

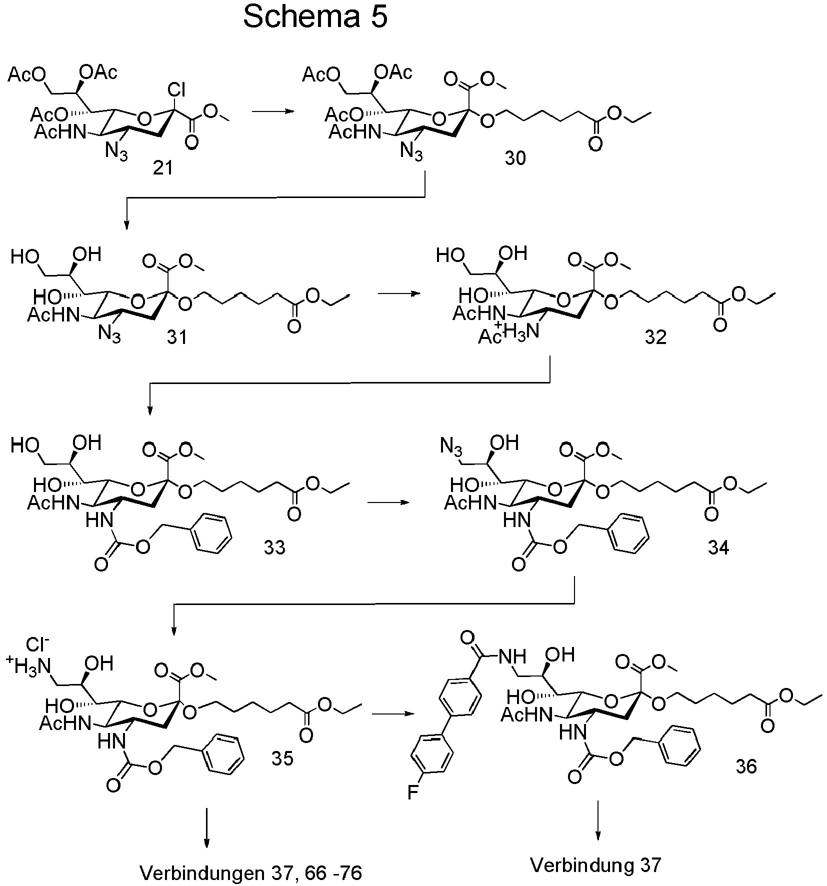

Die Verbindungen wurden wie im Folgenden beschrieben erhalten:
Alle verwendeten aber nicht beschriebenen Verbindungen wurden gekauft oder nach bekannten Literaturvorschriften hergestellt.

Für Reinigungen mit Kieselgel wurde Kieselgel Si60 43-60mm verwendet. Pro Gramm Substanz wurden ca. 100g Kieselgel benutzt. In Klammern angegeben ist das Laufmittel. Für Reinigungen über RP-18-Kieselgel (YMC CO LTD., YMC ODS-AQ) wurde dieses in Methanol suspendiert, in eine Säule gefüllt, mit Wasser vorgewaschen und die Substanz als Lösung oder Suspension mit Wasser aufgetragen. Das Säulenvolumen betrug ca. 5 cm in der Höhe und ca. 1 cm im Durchmesser. Das Lösungsmittel wurde mit geringem Druck, erzeugt durch einen handbetriebenen Druckball, durch die Säule gedrückt. Als Laufmittel wurde ein Gradient von Wasser zu Ethanol benutzt soweit nicht anders angegeben.

Lösungsmittel wurden mittels eines Vakuumrotationsverdampfers bei reduziertem Druck und einer Badtemperatur von 40°C entfernt. In den folgenden Synthesen ist dieser Arbeitsschritt als "Konzentration" benannt.

Alle Substanzen wurden aus Wasser oder einer Wasser-Dioxan Mischung lyophilisiert. Die Reaktionen und Substanzen wurden mit Dünnschichtchromatographie kontrolliert. Hierfür wurden kieselgelbeschichtete Aluminiumplatten mit Fluoreszenzindikator verwendet (Merck TLC Silica gel 60 F₂₅₄). Die Substanzdetektion erfolgte unter UV-Licht bei 366 und 254 nm. Die Chromatogramme wurden anschließend mit verdünnter Schwefelsäure besprüht und erhitzt um die Substanzen nachzuweisen. Amine wurden zum Nachweis mit Ninhydrin-Lösung besprüht und erhitzt. Einzelheiten und weitere Nachweisverfahren sind in "Anfärbereagenzien für Dünnschicht- und Papierchromatographie" Merck, 1970 erläutert. Spektroskopische Daten wurden mit Bruker ApexQe hybrid 9.4 T FT-ICR (ESI) und Varian 500MHz or 300MHz system (NMR) Geräten gemessen.

| Abkürzungen | |
|---|---|
| DMF | N,N-Dimethylformamid |
| DIPEA | N,N-Diisopropylethylamin |
| EtOH | Ethanol |
| HAc | Essigsäure |
| HATU | O-(7-Azabenzotriazol-1-yl)-N,N,N',N'-tetramethyluronium |
| MeOH | Methanol |
| TEA | Triethylamin |

### Verbindung 1

Hergestellt nach: Tetrahedron Letters, 1994, 50 (25), 7445-7460

### Verbindung 4

Eine Lösung von 2,2 g Verbindung 1 in 100 ml MeOH wurde mit 4,45 g Triphenylphosphin und 6 ml H₂O versetzt und die Suspension wurde 18 h bei Raumtemperatur gerührt. Unter Rühren wurden 20 ml 20%ige Essigsäure und 90 ml H₂O zugegeben, eine halbe Stunde gerührt, die Suspension auf 100 ml konzentriert, und dreimal mit 100 ml Dichlormethan ausgeschüttelt. Die wässrige Phase wurde lyophilisiert. Ausbeute: 2,67 g Feststoff

### Verbindung 3

Eine Lösung von 2,0 g Verbindung 2 in 20 ml DMF wurde mit 1.69 g 4-Biphenylcarbonsäure 4-nitrophenylester und 2.09 ml TEA gemischt und 17h gerührt. Die Lösung wurde konzentriert und der Rückstand über eine Kieselgelsäule (CHCl₃:MeOH Gradient) gereinigt. Ausbeute: 3,2 g

### Verbindung 4

Eine Lösung von 2,2 g Verbindung 3 in 20 ml Essigsäure und 20 ml Essigsäureanhydrid wurde bei 0°C mit 2 ml konzentrierter Schwefelsäure versetzt und 48h bei 25°C gerührt. Die Essigsäure wurde im Rotationsverdampfer entfernt und die verbleibende Lösung langsam in 1000 ml gesättigte Natriumhydrogencarbonat Lösung getropft. Die Suspension wurde 4h gerührt und dreimal mit 200 ml Ethylacetat extrahiert. Die vereinigten organischen Phasen wurden mit ges. NaCl Lösung gewaschen, getrocknet (MgSO₄), filtriert und konzentriert. Der Rückstand wurde über eine Kieselgelsäule (CH₂Cl₂:MeOH Gradient) gereinigt. Ausbeute: 2,0 g

### Verbindung 5

Eine Lösung von 1,25 g Verbindung 4 und 1,19 ml Trimethylsilylazid in 20 ml trockenem tert-Butanol wurde 4 h bei 80°C gerührt. Die Lösung wurde eingeengt und der Rückstand ohne weitere Reinigung weiter verwendet.

### Verbindung 6

Eine Lösung von 1350 mg Verbindung 5 in 0.8 ml Acetonitril und 2 ml Wasser wurde bei 60°C mit 538 mg N-lodsuccinimid gemischt, 30 min gerührt und konzentriert. Der Rückstand wurde über eine Kieselgelsäule (CHCl₃ to MeOH) gereinigt. Ausbeute: 1170 mg.

### Verbindung 7

Eine Lösung von 1140 mg Verbindung 6 in 10 ml trockenen Acetonitril wurde mit 0,277 ml 1,8-Diazabicyclo[5.4.0]undec-7-en gemischt, 15 min gerührt und konzentriert. Der Rückstand wurde über eine Kieselgelsäule (CHCl₃ zu MeOH) gereinigt. Ausbeute: 750 mg

### Verbindung 8

Eine Lösung von 20 mg Camphorsulfonsäure in 0,5 ml 6-Hydroxyhexansäureethylester wurde mit 150 mg Verbindung 7 Gemisch, 20 min gerührt und mit 30 ml CH₂Cl₂ verdünnt. Der Überschuss an 6-Hydroxyhexansäureethylester wurde über eine Kieselgelsäule entfernt (CH₂Cl₂:MeOH 100:1). Das Produkt wurde von der Säule eluiert (CH₂Cl₂:MeOH 10:1) und über RP18 gereinigt. Ausbeute: 50 mg

### Verbindung 9

Eine Lösung von 50 mg Verbindung 8 in 5 ml MeOH wurde mit 0.05 ml H₂O und 51 mg Triphenylphosphin gemischt und 17 h gerührt. Die Lösung wurde mit 0.5 ml Essigsäure (20%) und 10 ml H₂O versetzt und die Suspension wurde über RP18 (H₂O, dann verdünnte HCl pH3, dann H₂O zu EtOH Gradient) gereinigt. Ausbeute: 40 mg

### Verbindungen 10

Eine Lösung von 15 mg Verbindung 9 in 0.5 ml DMF wurde mit 33 ml DIPEA und 5.0 ml Propansäureanhydrid versetzt, 15 min gerührt, mit 2 ml H₂O verdünnt, mit 0.1 ml 2M NaOH versetzt und 2h gerührt. Die Lösung wurde mit 20% Essigsäure auf pH 8-9 eingestellt, mit 0.5 ml gesättigter Na₂CO₃ Lösung versetzt und über RP18 gereinigt. Ausbeute: 9 mg; ¹H NMR (500 MHz, CD₃OD): d ppm 7.94 (d, *J* = 8.63 Hz, 2H), 7.73 (d, *J* = 8.38 Hz, 2H), 7.68 (dd, *J* = 8.19, 1.17 Hz, 1 H), 7.47 (t, *J* = 7.66 Hz, 2H), 7.38 (t, *J* = 7.39 Hz, 1 H), 4.08 (ddd, *J* = 8.41, 7.81, 3.14 Hz, 1 H), 4.03 (dd, *J* = 10.86, 10.03 Hz, 1 H), 3.98 (t, *J* = 10.27 Hz, 1 H), 3.85 (td, *J* = 9.04, 6.85 Hz, 1 H), 3.81 (dd, *J* = 9.97, 1.89 Hz, 1 H), 3.78 (dd, *J* = 13.59, 3.02 Hz, 1 H), 3.59 (dd, *J* = 13.88, 7.80 Hz, 1 H), 3.55 (td, *J* = 9.19, 7.08 Hz, 1 H), 3.50 (d, *J* = 10.24 Hz, 1 H), 3.43 (dd, *J* = 9.00, 2.00 Hz, 1 H), 2.22 (q, *J* = 7.63 Hz, 2H), 2.17 (dd, *J* = 8.24, 7.03 Hz, 2H), 1.90 (s, 3H), 1.66-1.57 (m, 4H), 1.44-1.35 (m, 2H), 1.13 (t, *J* = 7.65 Hz, 3H); HRMS (ESI-neg) calcd for C33H41N3O12 [M-2Na+H]⁻: 672.2774, gefunden: 672.2801

### Verbindung 11

Eine Lösung von 24 mg Verbindung 9 in 1 ml CH₂Cl₂ wurde mit 21 µl TEA und 3.6 µl Methansulfonsäurechlorid versetzt und 15 min gerührt. Die Lösung wurde mit 2 ml gesättigter NaHCO₃ Lösung versetzt, 15 min gerührt mit 20 ml CH₂Cl₂ und 20 ml gesättigter NaHCO₃ versetzt. Die org. Phase wurde getrocknet (MgSO₄), filtriert und über RP18 gereinigt. Der Rückstand wurde in wenig EtOH gelöst. Die Lösung wurde mit Wasser versetzt bis eine Trübung auftrat, mit 2M NaOH auf pH 12-13 eingestellt, 2h gerührt, mit verdünnter Essigsäure neutralisiert und konzentriert. Der Rückstand wurde mit wenig Wasser gelöst, mit 0.3 ml gesättigter NaHCO₃ versetzt und über RP18 gereinigt. Ausbeute: 11 mg; ¹H NMR (500 MHz, CD₃OD): δ ppm 7.94 (d, *J* = 8.68 Hz, 2H), 7.73 (d, *J* = 8.70 Hz, 2H), 7.68 (dd, *J* = 8.41, 1.23 Hz, 2H), 7.47 (t, *J* = 7.62 Hz, 2H), 7.38 (t, *J* = 7.39 Hz, 1 H), 4.08 (ddd, *J* = 9.06, 7.59, 3.21 Hz, 1 H), 3.87 (t, *J* = 10.48 Hz, 1 H), 3.85 (td, *J* = 9.22, 6.53 Hz, 1 H), 3.77 (dd, *J* = 13.73, 3.14 Hz, 1 H), 3.75 (dd, *J* = 10.49, 2.06 Hz, 1 H), 3.59 (dd, *J* = 13.75, 7.59 Hz, 1 H), 3.54 (td, *J* = 9.08, 6.72 Hz, 1 H), 3.47 (d, *J* = 10.44 Hz, 1H), 3.44 (dd, *J* = 8.99, 1.98 Hz, 1H), 3.34 (t, *J* = 10.45 Hz, 1H), 3.05 (s, 3H), 2.16 (dd, *J* = 8.22, 7.14 Hz, 2H), 1.98 (s, 3H), 1.66-1.57 (m, 4H), 1.45-1.35 (m, 2H); HRMS (ESI-neg) calcd for C31H39N3O13S [M-2Na+H]⁻: 694.2287, gefunden: 694.2302

### Verbindungen 12

Eine Lösung von 70 mg Verbindung 5 in 10 ml Acetylchlorid wurde bei 0°C mit 0.5 g LiCl versetzt und 0.5 ml HCl (37%) wurde innerhalb 1h zugetropft. Die Lösung wurde 5h bei 0°C gerührt und mit LiCl bis zur Sättigung versetzt. Die Suspension wurde 5 Tage bei RT gerührt, wobei alle 20h auf 0°C gekühlt, 0.1 ml HCl (37%) zugetropft und anschließend 4h bei 0°C gerührt wurde. Die Suspension wurde konzentriert, der Rückstand mit CH₂Cl₂ versetzt, die Suspension mit kalter NaHCO₃ Lösung und mit ges. NaCl Lösung gewaschen, getrocknet (MgSO₄), filtriert und konzentriert. Der Rückstand wurde direkt ohne Lagerung eingesetzt.

### Verbindung 13

Eine Mischung von 2 ml CH₂Cl₂, 2 ml 6-Hydroxyhexansäureethylester und 0.5 g trockenes Molsieb A4 wurde mit 50 mg Verbindung 12 gemischt und 5 Tage gerührt. CH₂Cl₂ wurde im Vakuum entfernt, die Suspension 1h bei 40°C gerührt, mit CH₂Cl₂ verdünnt. 6-Hydroxyhexansäureethylester wurde über eine Kiesegelsäule (CH₂Cl₂:MeOH 100:1) entfernt, die Produkte von der Säule eluiert (CH₂Cl₂:MeOH 5:1) und konzentriert. Der Rückstand wurde über RP18 gereinigt. Ausbeute: 20 mg

### Verbindung 14

Eine Lösung von 20 mg Verbindung 13 in 5 ml MeOH und 0.2 ml HAc (20%) wurde mit einer katalytischen Menge Pd auf Kohle versetzt und 60 min hydriert. Die Suspension wurde über Celite filtriert, konzentriert und lyophilisiert. Ausbeute: 16 mg

### Verbindung 15

Eine Lösung von 16 mg Verbindung 14 in 1 ml DMF wurde mit 13 mg Schwefeltrioxid-Pyridin (1:1) und 58 µl TEA versetzt, 20 min gerührt, mit MeOH versetzt und weitere 20 min gerührt. Der Überschuss TEA wurde im Vakuum entfernt und die Lösung wurde mit Wasser verdünnt, mit ges. NaHCO₃ auf pH 8 eingestellt und über RP18 gereinigt. Das Produkt wurde mit wenig EtOH gelöst und die Lösung wurde mit Wasser bis zur Trübung verdünnt, mit 2M NaOH auf pH 13 eingestellt, 2h gerührt, mit 10ml Wasser verdünnt, mit verdünnter Essigsäure auf pH 8 eingestellt und über RP18 gereinigt. Ausbeute: 4 mg; ¹H NMR (500 MHz, CD₃OD): δ ppm 7.94 (d, *J* = 8.45 Hz, 2H), 7.73 (d, *J* = 8.53 Hz, 2H), 7.68 (dd, *J* = 8.33, 1.11 Hz, 2H), 7.47 (t, *J* = 7.68 Hz, 2H), 7.38 (t, *J* = 7.39 Hz, 1 H), 4.08 (ddd, *J* = 8.82, 7.94, 3.21 Hz, 1 H), 3.79 (td, *J* = 9.10, 6.96 Hz, 1 H), 3.78 (dd, *J* = 13.54, 3.23 Hz, 1 H), 3.71 (dd, *J* = 10.22, 1.90 Hz, 1 H), 3.61 (t, *J* = 10.20 Hz, 1 H), 3.58 (dd, *J* = 13.66, 7.75 Hz, 1 H), 3.47 (td, *J* = 9.05, 7.02 Hz, 1 H), 3.45 (dd, *J* = 9.05, 1.94 Hz, 1 H), 3.39 (ddd, *J* = 12.12, 10.61, 4.41 Hz, 1 H), 3.07 (dd, *J* = 12.68, 4.54 Hz, 1 H), 2.16-2.12 (m, 2H), 2.01 (s, 3H), 1.65-1.52 (m, 5H), 1.40-1.32 (m, 2H); HRMS (ESI-neg) calcd for C30H36N3Na3O13S [M-3Na+2H]⁻: 680.2131, gefunden: 680.2153

### Verbindung 16

Eine Lösung von 65 mg Verbindung 14 in 1 ml DMF wurde mit 12 µl Propansäureanhydrid und 35 µl TEA versetzt, 1h gerührt und konzentriert. Der Rückstand wurde über RP18 gereinigt. Ausbeute: 60 mg; ¹H NMR (500 MHz, CD₃OD): δ ppm 7.89 (d, *J* = 8.55 Hz, 2H), 7.65 (d, *J* = 8.56 Hz, 2H), 7.60 (dd, *J* = 8.30, 1.22 Hz, 2H), 7.45 (t, *J* = 7.54 Hz, 2H), 7.38 (t, *J* = 7.36 Hz, 1H), 7.10 (dd, *J* = 8.03, 4.48 Hz, 1H), 5.76 (d, *J* = 8.03 Hz, 1H), 5.69 (d, *J* = 9.33 Hz, 1 H), 5.31 (td, *J* = 9.69, 3.31 Hz, 1 H), 5.22 (dd, *J* = 9.73, 2.13 Hz, 1 H), 4.29 (ddd, *J* = 15.04, 8.20, 3.13 Hz, 1 H), 4.10 (q, *J* = 7.13 Hz, 2H), 4.10 (dd, *J* = 10.08, 2.24 Hz), 3.99-3.88 (m, 2H), 3.78 (td, *J* = 9.39, 6.28 Hz, 1H), 3.77 (s, 3H), 3.21 (td, *J* = 9.41, 6.39 Hz, 1H), 3.06 (td, *J* = 15.07, 4.12 Hz, 1H), 2.57 (dd, *J* = 12.82, 3.58 Hz, 1H), 2.29 (t, *J* = 7.73 Hz, 2H), 2.22 (s, 3H), 2.13 (s, 3H), 1.89 (s, 3H), 1.72 (t, *J* = 12.64 Hz, 1 H), 1.67-1.60 (m, 2H), 1.58-1.52 (m, 2H), 1.44-1.32 (m, 2H), 1.24 (t, *J* = 7.14 Hz, 3H), 1.09 (t, *J* = 7.60 Hz, 3H); HRMS (ESI-pos) calcd for C40H53N3O13 [M+Na]⁺: 806.3471, gefunden: 806.3492

### Verbindung 17

Eine Lösung von 30 mg Verbindung 16 in wenig EtOH wurde mit Wasser bis zur Trübung versetzt, mit 2M NaOH auf pH 12-13 eingestellt und 2h bei dem pH gehalten. Die Lösung wurde mit 10 ml Wasser verdünnt, mit verdünnter Essigsäure auf pH 8-9 eingestellt und über RP18 gereinigt. Ausbeute: 24 mg; ¹H NMR (500 MHz, CD₃OD): δ ppm 7.94 (d, *J* = 8.61 Hz, 2H), 7.72 (dd, *J* = 8.69, 1.85 Hz, 2H), 7.67 (dd, *J* = 6.95, 1.46 Hz, 2H), 7.46 (t, *J* = 7.61 Hz, 2H), 7.38 (t, *J* = 7.39 Hz, 1 H), 4.11-4.02 (m, 2H), 3.84-3.76 (m, 4H), 3.60 (dd, *J* = 13.57, 7.35 Hz, 1 H), 3.51-3.44 (m, 2H), 2.67 (dd, *J* = 12.43, 4.40 Hz, 1 H), 2.20-2.12 (m, 4H), 1.91 (s, 3H), 1.66 (t, *J* = 12.56 Hz, 1 H), 1.64-1.53 (m, 4H), 1.41-1.33 (m, 2H), 1.11 (t, *J* = 7.66 Hz, 3H); HRMS (ESI-neg) calcd for C33H41N3Na2O11[M-2Na+H]⁻: 656.2825, gefunden: 656.2815

### Verbindung 18

Eine Lösung von 20 mg Verbindung 14 in 1 ml DMF wurde mit 5 mg Bersteinsäureanhydrid und 21 µl TEA versetzt, 30 min gerührt, konzentriert. Der Rückstand wurde über RP18 gereinigt. Ausbeute: 17 mg; ¹H NMR (300 MHz, CD₃OD): δ ppm 7.84 (d, *J* = 8.36 Hz, 2H), 7.71 (d, *J* = 8.26 Hz, 2H), 7.65 (d, *J* = 7.46 Hz, 2H), 7.46 (t, *J* = 7.45 Hz, 2H), 7.37 (t, *J* = 7.27 Hz, 1 H), 5.44 (ddd, *J* = 9.36, 6.87, 2.61 Hz, 1 H), 5.26 (dd, *J* = 9.25, 2.13 Hz, 1 H), 4.20 (dd, *J* = 10.13, 1.95 Hz, 1 H), 4.09 (q, *J* = 7.11 Hz, 2H), 3.95-3.73 (m, 7H), 3.37 (dd, *J* = 14.53, 6.89 Hz, 1 H), 3.23 (td, *J* = 9.56, 6.28 Hz, 1 H), 2.44 (dd, *J* = 13.07, 2.97 Hz, 1H), 2.29 (t, *J* = 7.39 Hz, 2H), 2.17 (s, 2H), 2.12 (s, 2H), 1.85 (s, 3H), 1.75 (t, *J* = 12.75 Hz, 1 H), 1.67-1.47 (m, 4H), 1.45-1.30 (m, 4H), 1.22 (t, *J* = 7.12 Hz, 3H); HRMS (ESI-neg) calcd for C41H52N3NaO15 [M-Na]⁻: 826.3404, gefunden: 826.3381

### Verbindung 20

Eine Lösung von 25 mg Verbindung 19 (WO 2013097942) in Wasser wurde mit 2M NaOH auf pH 13 eingestellt, 2h bei diesem pH gehalten, mit verdünnter HAc auf pH 8 eingestellt und über RP18 gereinigt. Das lyophilisierte Produkt (20 mg) wurde in 0.5 ml DMF gelöst und portionsweise mit Schwefeltrioxid-Pyridin (1:1) versetzt bis nur noch ca. 20% Edukt im DC detektierbar waren. MeOH wurde zugegeben, 30 min gerührt und das MeOH im Vakuum entfernt. Die Lösung wurde mit Wasser verdünnt, mit NaHCO₃ Lösung auf pH 8 eingestellt und über RP18 gereinigt. Ausbeute: 5 mg (21%) ¹H NMR (500 MHz, CD₃OD): δ ppm 7.94 (d, *J* = 8.63 Hz, 2H), 7.73 (d, *J* = 8.62 Hz, 2H), 7.68 (dd, *J* = 8.38, 1.21 Hz, 2H), 7.47 (t, *J* = 7.66 Hz, 2H), 7.38 (t, *J* = 7.40 Hz, 1H), 4.46 (ddd, *J* = 11.61, 9.99, 5.15 Hz, 1 H), 4.09 (ddd, *J* = 8.84, 7.74, 3.27 Hz, 1 H), 3.87 (td, *J* = 9.47, 6.40 Hz, 1 H), 3.81 (t, *J* = 10.23 Hz, 1 H), 3.79 (dd, *J* = 13.65, 3.28 Hz, 1 H), 3.73 (dd, *J* = 10.44, 1.89 Hz, 1 H), 3.58 (dd, *J* = 13.68, 7.55 Hz, 1H), 3.58 (td, *J* = 9.49, 6.34 Hz, 1H), 3.46 (dd, *J* = 8.96, 1.86 Hz, 1 H), 3.19 (dd, *J* = 12.26, 5.10 Hz, 1 H), 2.73 (dd, *J* = 8.03, 7.62 Hz, 2H), 2.59 (dt, *J* = 7.14, 5.04 Hz, 2H), 2.40 (dd, *J* = 8.02, 7.60 Hz, 2H), 2.00 (s, 3H), 1.85-1.79 (m, 2H), 1.71 (t, *J* = 12.00 Hz, 1 H); HRMS (ESI-neg) calcd for C30H35N2Na3O14S2 [M-3Na+2H]⁻: 713.1692, gefunden: 713.1715

### Verbindung 22

Verbindung 21 (3500 mg) wurde in 20 ml trockenem Allylalkohol gelöst, mit 5 g gemahlenem Molsieb A4 versetzt und 5h gerührt. Die Suspension wurde bei 40°C konzentriert, der Rückstand mit CH₂Cl₂ versetzt, über Celite filtriert und über eine Kieselgelsäule gereinigt (CH₂Cl₂ zu MeOH). Das isolierte Produkt wurde aus Ethylacetat / Diethylether kristallisiert. Ausbeute: 1600 mg

### Verbindung 23

Eine Lösung von 1600 mg Verbindung 22 in 20 ml trockenem MeOH wurde mit 1 ml 1 M Natriummethanolat versetzt. Nach vollständiger Reaktion wurde die Lösung mit Ionenaustauscher Harz (Dowex H+Form) in MeOH neutralisiert. Das Harz wurde abfiltriert und die Lösung eingeengt. Ausbeute: 1200 mg

### Verbindung 24

Eine Lösung von 1200 mg Verbindung 23 in 20 ml MeOH und 4 ml Wasser wurde mit 2010 mg Zinkpulver und 1670 mg Ammoniumchlorid versetzt und 5h gerührt. Die Suspension wurde über Celite filtriert, eingeengt und direkt weiter eingesetzt. Ausbeute: 1200 mg

### Verbindung 25

Eine Lösung von 1200 mg Verbindung 24 in 20 ml MeOH wurde mit 721 mg Boc-Anhydrid und 0.49 ml TEA versetzt, 2h gerührt und eingeengt. Der Rückstand wurde über Kieselgel (Ethylacetat) gereinigt. Ausbeute: 976 mg

### Verbindung 26

Verbindung 25 (976 mg) wurde dreimal in 10 ml trockenem DMF gelöst und im Vakuum konzentriert. Der wasserfreie Rückstand wurde in 10 ml trockenem DMF gelöst und bei 0°C mit 517 mg trockenem Lithiumazide, 1678 mg Tetrabrommethan und 664 mg Triphenylphosphin versetzt. Die Lösung wurde 16h bei 25°C gerührt, mit 5 ml MeOH versetzt und konzentriert. Der Rückstand wurde mit Wasser versetzt und zweimal mit Ethylacetat extrahiert. Die vereinigten organischen Phasen wurden mit gesättigtem NaCl gewaschen, getrocknet (MgSO₄), filtriert und über eine Kieselgelsäule (Ethylacetat) gereinigt. Ausbeute: 700 mg

### Verbindung 27

Eine Lösung von 700 mg Verbindung 26 in 50 ml DMF wurde mit 0.5 ml Wasser, 1130 mg Triphenylphosphin und 1375 mg 4-Biphenylcarbonsäure-4-nitrophenylester versetzt, drei Tage gerührt, eingeengt und über eine Kieselgelsäule (CH₂Cl₂ zu MeOH) gereingt. Ausbeute: 440 mg

### Verbindung 28

Eine Lösung von 30 mg Verbindung 27 in 5 ml MeOH und 2 ml Wasser wurde 20 min mit Stickstoff gespült, mit 41 µl Thiopropionsäure und einer katalytischen Menge Azaisobutyronitril versetzt und 12h mit UV-Licht bestrahlt. Die Lösung wurde konzentriert und der Rückstand über RP18 gereinigt. Ausbeute: 30 mg; ¹H NMR (500 MHz, CD₃OD): δ ppm 7.93 (d, *J* = 8.62 Hz, 2H), 7.71 (d, *J* = 8.65 Hz, 2H), 7.66 (dd, *J* = 8.38, 1.21 Hz, 2H), 7.45 (t, *J* = 7.62 Hz, 2H), 7.37 (t, *J* = 7.39 Hz, 1 H), 4.05 (ddd, *J* = 8.87, 7.28, 3.21 Hz, 1 H), 3.91-3.82 (m, 6H), 3.80 (dd, *J* = 13.83, 3.23 Hz, 1 H), 3.67-3.62 (m, 1 H), 3.60 (dd, *J* = 13.78, 7.35 Hz, 1 H), 3.49 (td, *J* = 9.37, 6.42 Hz, 1 H), 3.45 (dd, *J* = 8.83, 1.25 Hz, 1 H), 2.71 (dd, *J* = 8.46, 7.06 Hz, 2H), 2.62-2.51 (m, 3H), 2.39 (dd, *J* = 8.56, 6.84 Hz, 2H), 1.90 (s, 3H), 1.82-1.70 (m, 3H), 1.42 (s, 9H); HRMS (ESI-neg) calcd for C36H48N3NaO12S [M-Na]⁻: 746.2964, gefunden: 746.2928

### Verbindung 29

Eine Lösung von 30 mg Verbindung 28 in wenig MeOH wurde mit Wasser versetzt bis eine Trübung auftrat, mit 2M NaOH auf pH 13 eingestellt, 2h bei dem pH gerührt, mit verdünnter Essigsäure auf pH 8-9 eingestellt und über RP18 gereinigt. Ausbeute: 25 mg; ¹H NMR (500 MHz, CD₃OD): δ ppm 7.93 (d, *J* = 8.61 Hz, 2H), 7.71 (d, *J* = 8.63 Hz, 2H), 7.66 (dd, *J* = 8.37, 1.19 Hz, 2H), 7.45 (t, *J* = 7.63 Hz, 2H), 7.36 (t, *J* = 7.39 Hz, 1 H), 4.06 (ddd, *J* = 8.96, 7.60, 3.17 Hz, 1 H), 3.87 (td, *J* = 9.48, 6.39 Hz, 1 H), 3.80-3.66 (m, 4H), 3.57 (dd, *J* = 13.22, 6.98 Hz, 1 H), 3.54 (td, *J* = 9.48, 6.25 Hz, 1 H), 3.44 (dd, *J* = 8.99, 1.24 Hz, 1 H), 2.72 (dd, *J* = 8.51, 6.99 Hz, 12H), 2.68 (dd, *J* = 12.56, 3.65 Hz, 1 H), 2.63-2.52 (m, 2H), 2.40 (dd, *J* = 8.51, 7.12 Hz, 2H), 1.92 (s, 3H), 1.83-1.76 (m, 2H), 1.61 (t, *J* = 12.16 Hz, 1 H), 1.42 (s, 9H); HRMS (ESI-neg) calcd for C36H45N3Na2O12S [M-2Na+H]⁻: 732.2808, gefunden: 732.2763

### Verbindung 30

Eine Lösung von 3500 mg Verbindung 21 in 4 ml CH₂Cl₂ wurde mit 1.9 ml 6-Hydroxyhexansäureethylester und 5 g trockenem Molsieb A4 verrührt und CH₂Cl₂ wurde im Rotationsverdampfer entfernt. Die Suspension wurde 6h bei 40°C gerührt, mit CH₂Cl₂ verdünnt und über Celite filtriert. 6-Hydroxyhexansäureethylester wurde über eine Kieselgelsäule (CH₂Cl₂:MeOH 100:1) entfernt. Die Produkte wurden mit (CH₂Cl₂:MeOH 5:1) von der Säule eluiert und erneut über Kieselgel gereinigt (CH₂Cl₂ zu MeOH). Ausbeute 2300 mg unsauberes Produkt welches ohne weitere Aufreinigung weiter eingesetzt wurde.

### Verbindung 31

Eine Lösung von 2300 mg Verbindung 30 in 20 ml trockenem MeOH wurde mit frischem Natriummethanolat in MeOH versetzt bis pH-Indikatorpapier pH 8-9 anzeigte. Nach 1h h wurde die Lösung mit Ionenaustauscher Harz (Dowex H+Form) in MeOH neutralisiert. Das Harz wurde abfiltriert, die Lösung eingeengt und der Rückstand über eine Kieselgelsäule (CH₂Cl₂ zu MeOH) gereinigt. Die Produkt enthaltenden Fraktionen wurden über RP18 gereinigt. Ausbeute: 374 mg

### Verbindung 32

Eine Lösung von 370 mg Verbindung 31 in 10 ml MeOH wurde mit 0.5 ml Essigsäure (20%) und einer katalytischen Menge Pd auf Kohle versetzt und 2h hydriert. Die Suspension wurde über Celite filtriert, die Lösung eingeengt und der Rückstand lyophilisiert. Ausbeute: 395 mg

### Verbindung 33

Eine Lösung von 395 mg Verbindung 32 in 10 ml DMF wurde mit 142 mg Benzyloxycarbonylchlorid und 0.31 ml TEA versetzt, 2h gerührt und eingeengt. Der Rückstand wurde über eine Kieselgelsäule (CH₂Cl₂ zu MeOH) gereinigt. Ausbeute: 245 mg

### Verbindung 34

Verbindung 33 (245 mg) wurde dreimal in 10 ml trockenem DMF gelöst und im Vakuum konzentriert. Der wasserfreie Rückstand wurde in 10 ml trockenem DMF gelöst und bei 0°C mit 100 mg trockenem Lithiumazide, 325 mg Tetrabrommethan und 139 mg Triphenylphosphin versetzt. Die Lösung wurde 16h bei 25°C gerührt, mit 5 ml MeOH versetzt und konzentriert. Der Rückstand wurde mit Wasser versetzt, zweimal mit Toluol und zweimal mit Ethylacetat extrahiert. Die vereinigten Ethylacetat Phasen wurden getrocknet (MgSO₄), filtriert und über RP18 gereinigt. Ausbeute: 176 mg

### Verbindung 35

Eine Lösung von 175 mg Verbindung 34 in 10 ml MeOH und 2 ml Wasser wurde mit 182 mg Zinkpulver und 151 mg Ammoniumchlorid versetzt und 2h gerührt. Die Suspension wurde über Celite filtriert, eingeengt und direkt weiter eingesetzt. Ausbeute: 270 mg

### Verbindung 36

Eine Lösung von 55 mg Verbindung 35 und 28 mg 4-(4-Fluorphenyl)-benzoesäure in 0.5 ml DMF wurde mit 49 mg O-(7-Azabenzotriazol-1-yl)-N,N,N',N'-tetramethyluronium und 0.146 ml DIPEA versetzt. Die Lösung wurde 10min gerührt, eingeengt und der Rückstand über RP18 gereinigt. Ausbeute 40 mg

### Verbindung 37

Eine Lösung von 40 mg Verbindung 36 in 5 ml MeOH und 0.5 ml HAc (20%) wurde mit einer katalytischen Menge Pd auf Kohle versetzt und 60 min hydriert. Die Suspension wurde über Celite filtriert, konzentriert und lyophilisiert. Der Rückstand (30 mg) wurde in 2 ml DMF gelöst und mit 20 mg Schwefeltrioxid-Pyridin (1:1) und 59 µl TEA versetzt,. Die Lösung wurde 60 min gerührt, mit 5 ml Wasser und 0.5 ml 2M NaOH versetzt und weitere 40 min gerührt, mit HAc neutralisiert und eingeengt. Der Rückstand wurde in Waser gelöst, mit 1 ml ges. Na₂CO₃ versetzt und über RP18 gereinigt. Ausbeute: 8 mg; ¹H NMR (500 MHz, CD₃OD): δ ppm 7.92 (d, *J* = 8.54 Hz, 2H), 7.71-7.67 (m, 4H), 7.19 (t, *J* = 8.84 Hz, 2H), 4.06 (ddd, *J* = 9.19, 8.27, 3.20 Hz, 1 H), 3.80-3.73 (m, 2H), 3.69 (dd, *J* = 10.20, 1.94 Hz, 1 H), 3.60 (dd, *J* = 10.84, 9.69 Hz, 1 H), 3.56 (dd, *J* = 13.66, 7.80 Hz, 1 H), 3.46 (td, *J* = 8.94, 6.86 Hz, 1 H), 3.43 (dd, *J* = 8.96, 1.93 Hz, 1 H), 3.37 (ddd, *J* = 12.25, 10.61, 4.49 Hz, 1 H), 3.05 (dd, *J* = 12.71, 4.53 Hz, 1H), 2.13 (dd, *J* = 8.02, 7.36 Hz, 2H), 2.00 (s, 3H), 1.62-1.51 (m, 5H), 1.38-1.31 (m, 2H); HRMS (ESI-neg) calcd for C30H35FN3Na3O13S [M-2Na+H]⁻: 720.1889, gefunden: 720.1856

### Verbindungen 38, 39, 40, 41, 42

Allgemeine Vorschrift: Zu einer Lösung von 0.20 mg Verbindung 9 in 50 µl DMF wurde ein 25facher Überschuss des entsprechenden Anhydrides und ein 100facher Überschuss DIPEA zugegeben. Die Lösung wurde 10min bei 20°C gerührt. Die Reaktion wurde per Dünnschichtchromatographie kontrolliert und war bei allen Ansätzen quantitativ. Die Lösung wurde mit 500 µl Wasser verdünnt, mit 50 µl 2M NaOH versetzt und nach 2h mit 50 µl HAc (20%) versetzt und lyophilisiert. Die Reaktion wurde nach der Zugabe von Essigsäure per Dünnschichtchromatographie kontrolliert und verlief bei allen Ansätzen quantitativ.

### Verbindungen 43, 44, 45, 46, 47, 48, 49, 50, 51, 52, 53, 54, 55, 56, 57, 58, 59, 60, 61, 62, 63, 64

Allgemeine Vorschrift: Zu einer Lösung von 0.20 mg Verbindung 9 in 50 µl DMF wurde ein 25facher Überschuss der entsprechenden Carbonsäure, ein 15facher Überschuss an HATU und ein 100facher Überschuss an DIPEA zugegeben und die Lösung wurde 10min bei 20°C gerührt. Die Reaktion wurde per Dünnschichtchromatographie kontrolliert und war bei allen Ansätzen quantitativ. Die Lösung wurde mit 500 µl Wasser verdünnt, mit 50 µl 2M NaOH versetzt und nach 2h mit 50 µl HAc (20%) versetzt und lyophilisiert. Die Reaktion wurde nach der Zugabe von Essigsäure per Dünnschichtchromatographie kontrolliert und verlief bei allen Ansätzen quantitativ.

### Verbindungen 65

Zu einer Lösung von 0.20 mg Verbindung 9 in 50 µl DMF wurde ein 25facher Überschuss Dansylchlorid und ein 100facher Überschuss an DIPEA zugegeben und die Lösung wurde 10min bei 20°C gerührt. Die Reaktion wurde per Dünnschichtchromatographie kontrolliert und war quantitativ. Die Lösung wurde mit 500 µl Wasser verdünnt, mit 50 µl 2M NaOH versetzt und nach 2h mit 50 µl HAc (20%) versetzt und lyophilisiert. Die Reaktion wurde nach der Zugabe von Essigsäure per Dünnschichtchromatographie kontrolliert und verlief quantitativ.

### Verbindungen 66, 67, 68, 69, 70, 71, 72, 73, 74, 75, 76

Allgemeine Vorschrift: Zu einer Lösung von 0.20 mg Verbindung 35 in 50 µl DMF wurde ein 25facher Überschuss der entsprechenden Carbonsäure, ein 15facher Überschuss an HATU und ein 100facher Überschuss an DIPEA zugegeben und die Lösung wurde 10min bei 20°C gerührt. Die Reaktionen wurden per Dünnschichtchromatographie kontrolliert und waren bei allen Ansätzen quantitativ. Die Lösung wurde mit 500 µl Wasser verdünnt, mit 50 µl 2M NaOH versetzt und nach 2h mit 50 µl HAc (20%) versetzt und lyophilisiert. Die Reaktion wurde nach der Zugabe von Essigsäure per Dünnschichtchromatographie kontrolliert und verlief bei allen Ansätzen quantitativ.

### Verbindung 77

Eine Lösung von 40 mg Verbindung 27 in 5 ml MeOH und 2 ml Wasser wurde 20 min mit Stickstoff gespült, mit 705 mg Cysteamin Hydrochlorid und einer katalytischen Menge Azaisobutyronitril versetzt und 12 h mit UV-Licht bestrahlt. Die Lösung wurde konzentriert und der Rückstand über RP18 gereinigt. Ausbeute: 407 mg; ¹H NMR (500 MHz, CD₃OD): δ ppm 7.93 (d, *J* = 8.64 Hz, 2H), 7.72 (d, *J* = 8.66 Hz, 2H), 7.66 (dd, *J* = 8.38, 1.23 Hz, 2H), 7.46 (t, *J* = 7.60 Hz, 2H), 7.38 (t, *J* = 7.38 Hz, 1 H), 4.07 (ddd, *J* = 8.67, 7.73, 3.08 Hz, 1 H), 3.93 (dd, *J* = 13.76, 2.91 Hz, 1 H), 3.92 (ddd, *J* = 9.63, 7.03, 5.02 Hz, 1 H), 3.88-3.84 (m, 1 H), 3.86 (s, 3H), 3.82 (dd, *J* = 10.34, 1.28 Hz, 1 H), 3.47 (dd, *J* = 8.76, 1.42 Hz, 1 H), 3.47-3.43 (m, 1 H), 3.43 (dd, *J* = 13.83, 7.64 Hz, 1 H), 3.14 (t, *J* = 6.96 Hz, 1 H), 3.14 (t, *J* = 6.56 Hz, 1 H), 2.80 (t, *J* = 6.75 Hz, 2H), 2.64 (t, *J* = 6.97 Hz, 2H), 2.55 (dd, *J* = 12.94, 4.00 Hz, 1 H), 1.93 (s, 3H), 1.86-1.71 (m, 3H), 1.43 (s, 9H); HRMS (ESI-neg) calcd for C36H48N3NaO12S [M-Na]⁻: 719.3320, gefunden: 719.3335

### Biologische Tests

Die biologische Aktivität der Substanzen wurde in einem ELISA basierten Inhibitionsassay wie in "Eur. J. Immunol. 2012, 42, 2792-2802" beschrieben bestimmt. Die Sialinsäurederivate der Formel (I) inhibieren die Bindung von löslichem CD22 an immobilisiertes IgM proportional zu ihrer Affinität zu CD22. Das lösliche CD22 Protein wurde in der Zelline CHO-Lec1 exprimiert und isoliert wie beschrieben "Eur. J. Immunol. 2012, 42, 2792-2802". Die bekannte Substanz "BPCNeu5Ac" (J. Exp. Med. 2002, 195, 1207-1213) wurde als Referenz in den Testen mitgetestet. Die Affinitätssteigerung ist als rIP (relative Inhibitorische Affinität) angegeben.

In Tabelle I die Affinität bzw. die relative Inhibitorische Potenz (rIP) der Sialinsäurederivate der Formel (I) gezeigt.

**Tabelle I**

| Nr. | Struktur | IC50 microM | rIP |
|---|---|---|---|
| BPC Neu5 Ac | 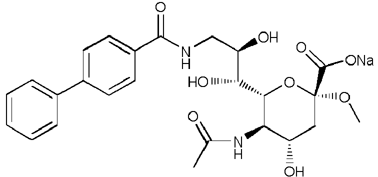 | 6.1 | 1 |
| 10 | 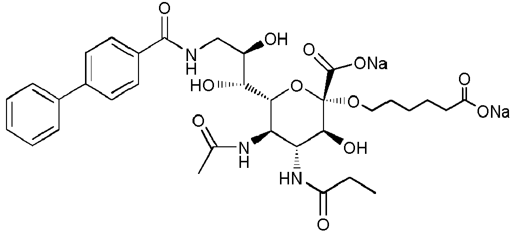 | 0.0060 | 1014 |
| 11 | 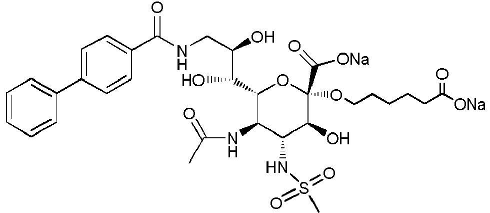 | 0.0077 | 793 |
| 15 | 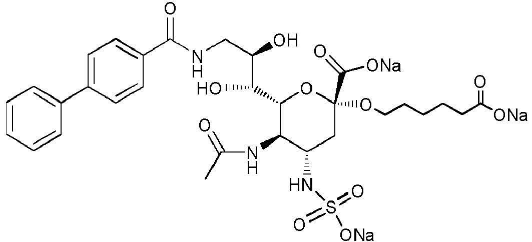 | 0.0032 | 1920 |
| 17 | 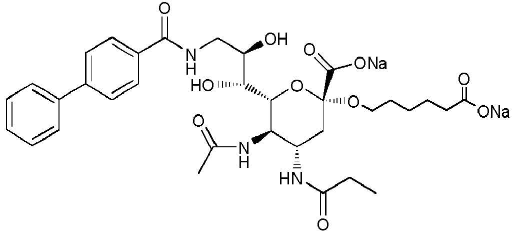 | 0.012 | 502 |
| 20 | 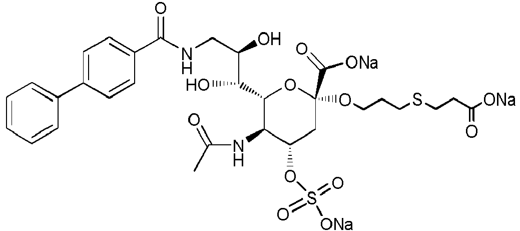 | 0.0020 | 3130 |
| 29 | 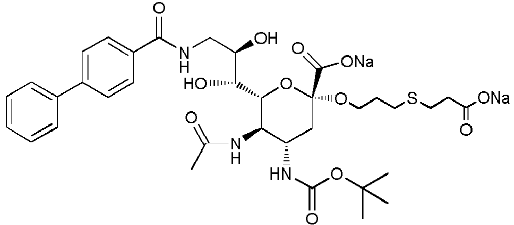 | 0.085 | 71 |
| 37 | 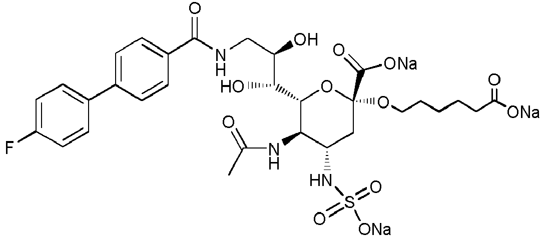 | 0.015 | 405 |

Weitere Sialinsäurederivate der Formel (I), bei denen rIP-Werte bestimmt wurden sind in Tabelle II aufgeführt. Als Referenz wurde die Verbindung 10, ein Sialinsäurederivat der Formel (I) mit überraschend hoher Affinität, im selben Test mitgetestet. Die Sialinsäurederivate der Formel (I) in Tabelle II zeigen eine mit der Referenzverbindung 10 vergleichbar hohe Affinität.

**Tabelle II**

| Nr. | Struktur | rIP |
|---|---|---|
| 10 | 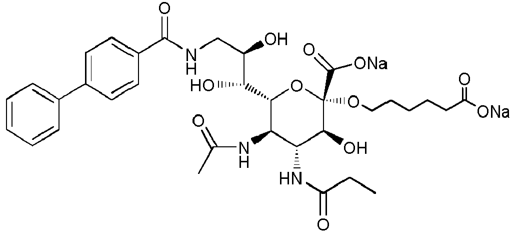 | 1 |
| 38 | 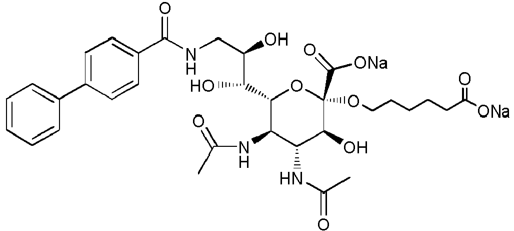 | 0.45 |
| 39 | 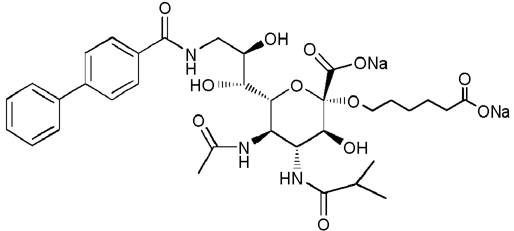 | 0.70 |
| 40 | 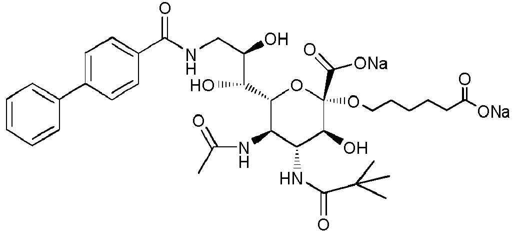 | 0.38 |
| 41 | 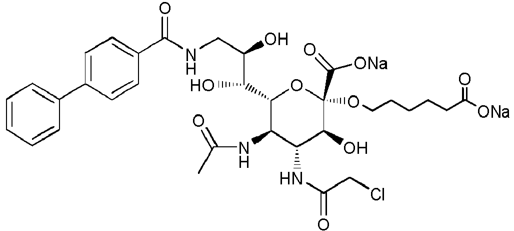 | 0.37 |
| 42 | 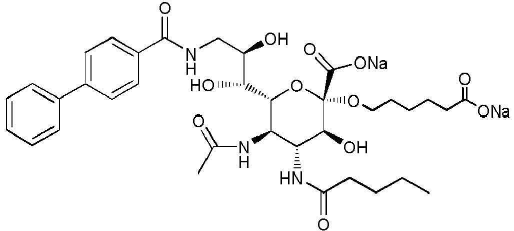 | 1.22 |
| 43 | 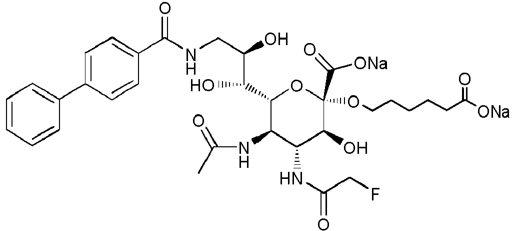 | 0.25 |
| 44 | 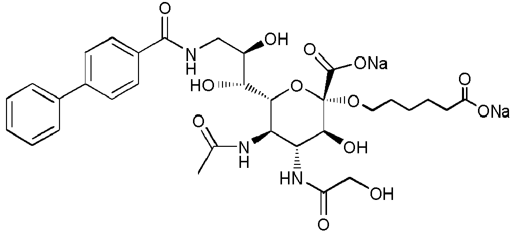 | 0.24 |
| 45 | 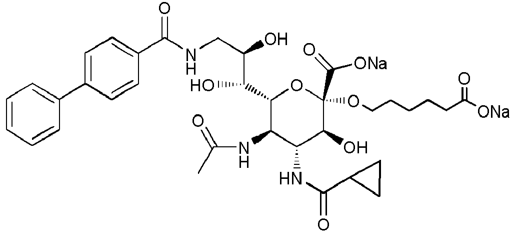 | 0.81 |
| 46 | 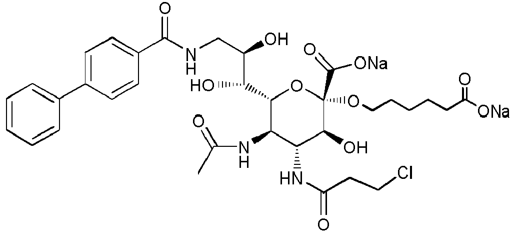 | 0.84 |
| 47 | 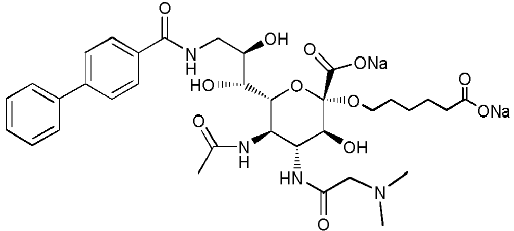 | 0.18 |
| 48 | 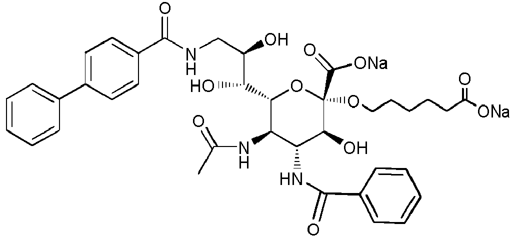 | 0.93 |
| 49 | 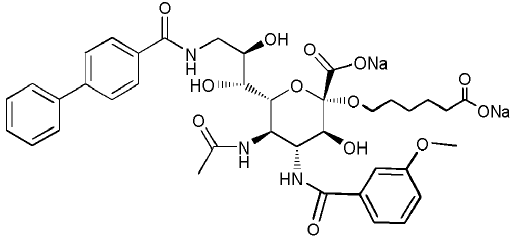 | 0.34 |
| 50 | 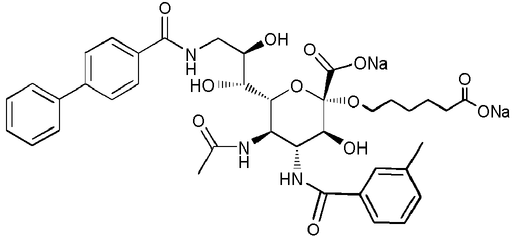 | 0.51 |
| 51 | 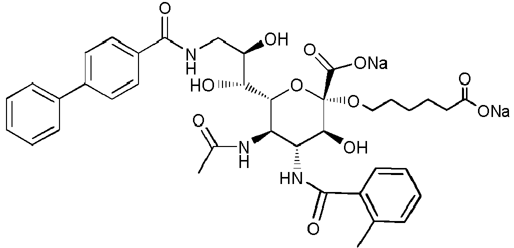 | 0.13 |
| 52 | 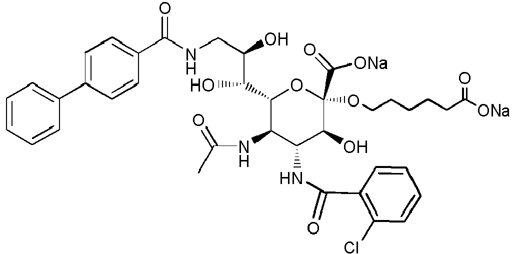 | 0.12 |
| 65 | 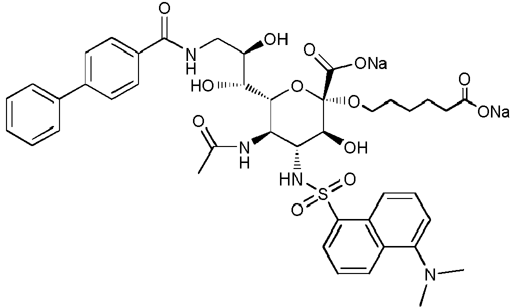 | 0.17 |

Weitere Sialinsäurederivate der Formel (I), bei denen rIP-Werte bestimmt wurden sind in Tabelle III aufgeführt. Als Referenz wurde die Verbindung 17, ein Sialinsäurederivat der Formel (I) mit überraschend hoher Affinität, im selben Test mitgetestet. Die Sialinsäurederivate der Formel (I) in Tabelle III zeigen eine mit der Referenzverbindung 17 vergleichbar hohe Affinität.

**Tabelle III**

| Nr. | Struktur | rIP |
|---|---|---|
| 17 | 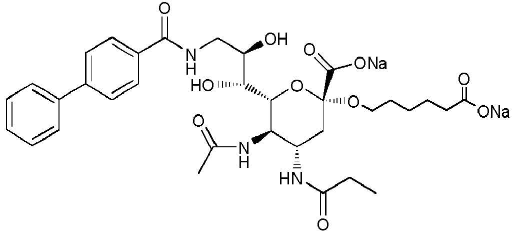 | 1 |
| 53 | 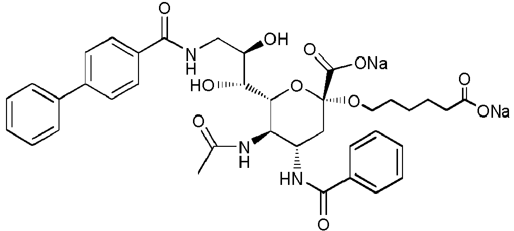 | 1.1 |
| 54 | 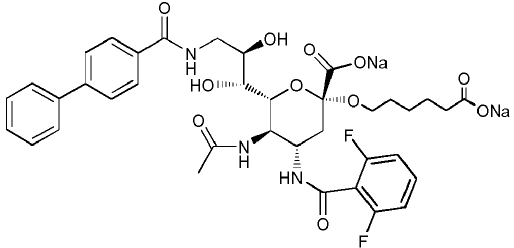 | 0.75 |
| 55 | 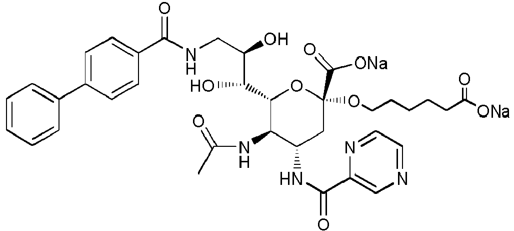 | 2.7 |
| 56 | 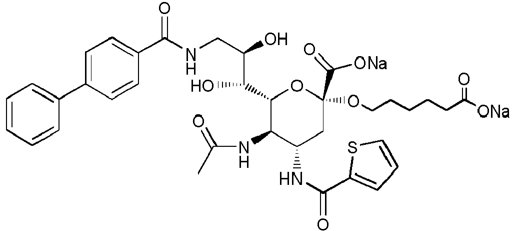 | 3.6 |
| 57 | 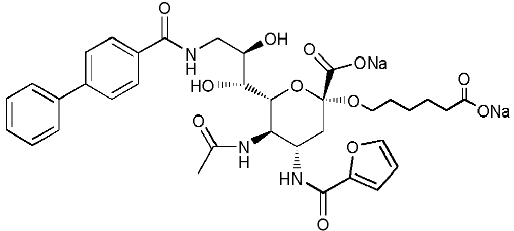 | 6.1 |
| 58 | 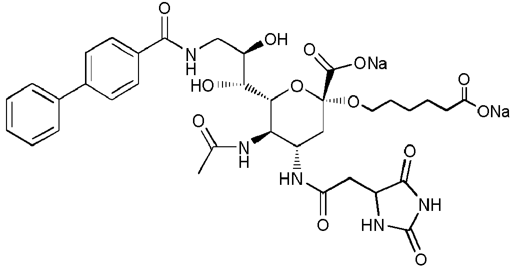 | 3.4 |
| 59 | 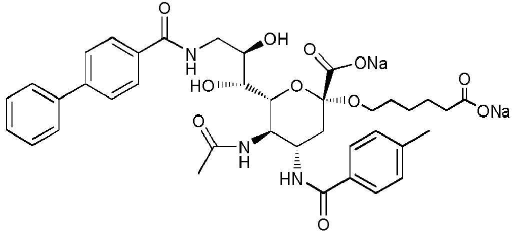 | 0.24 |
| 60 | 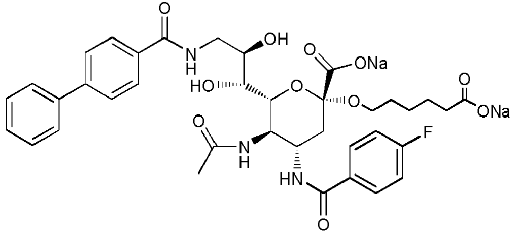 | 2.1 |
| 61 | 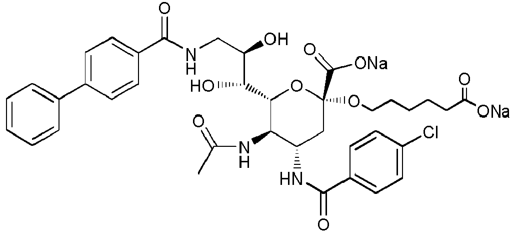 | 3.4 |
| 62 | 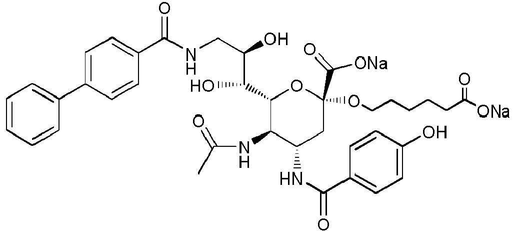 | 1.7 |
| 63 | 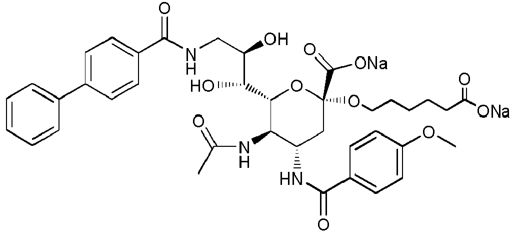 | 0.27 |
| 64 | 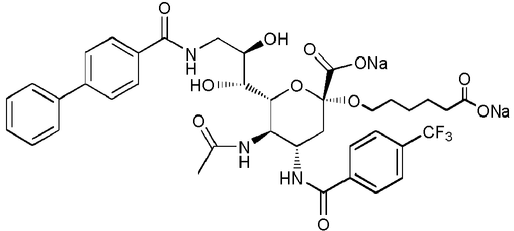 | 1.2 |
| 66 | 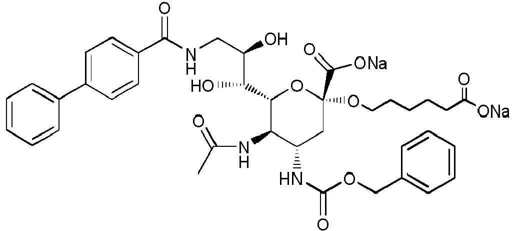 | 1.3 |

Weitere Sialinsäurederivate der Formel (I), bei denen rIP-Werte bestimmt wurden sind in Tabelle IV aufgeführt. Als Referenz wurde die Verbindung 66 im selben Test mitgetestet. Die Sialinsäurederivate der Formel (I) in Tabelle IV zeigen eine mit der Referenzverbindung 66 vergleichbar hohe Affinität.

**Tabelle IV**

| Nr. | Struktur | rIP |
|---|---|---|
| 66 | 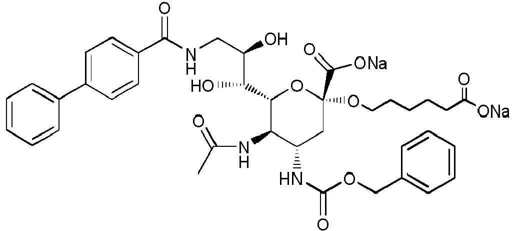 | 1 |
| 67 | 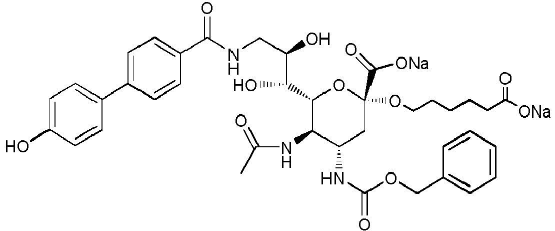 | 1.0 |
| 68 | 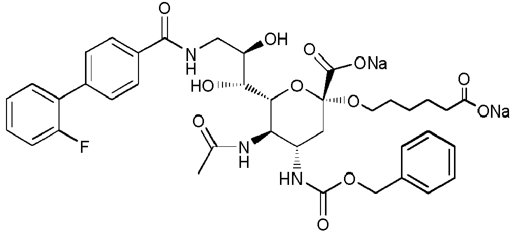 | 0.76 |
| 69 | 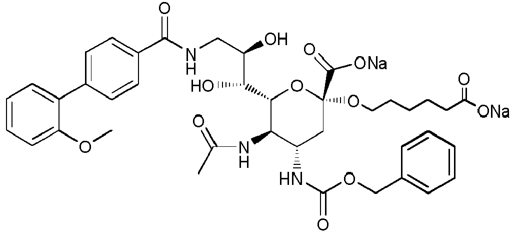 | 0.83 |
| 70 | 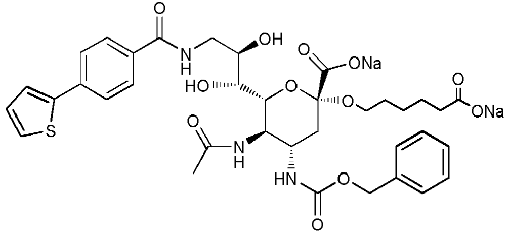 | 0.88 |
| 71 | 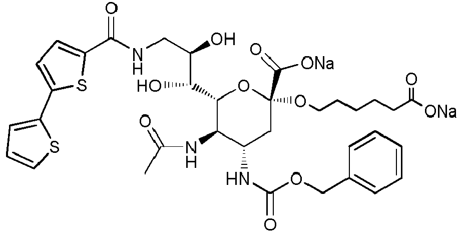 | 0.28 |
| 72 | 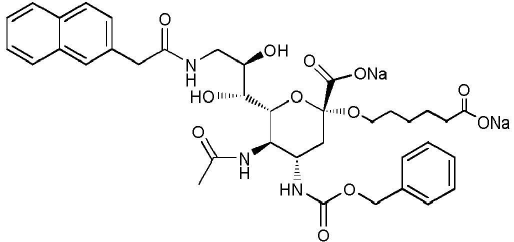 | 0.91 |
| 73 | 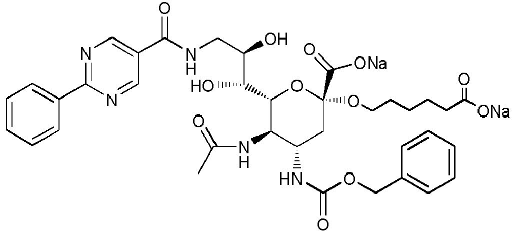 | 0.23 |
| 74 | 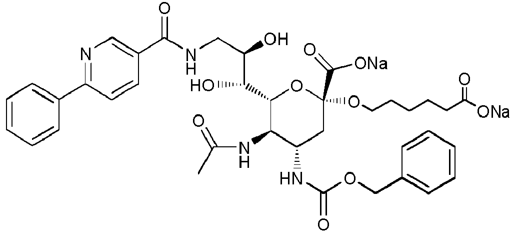 | 0.20 |
| 75 | 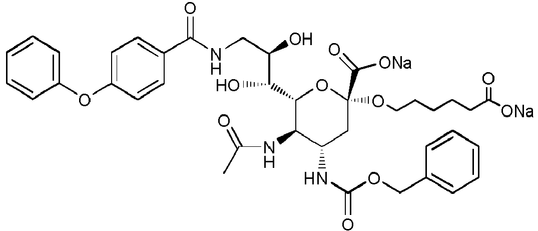 | 0.25 |
| 76 | 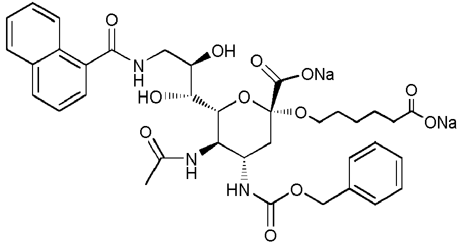 | 0.35 |

In Tabelle V sind Sialinsäurederivate der Formel (I) gezeigt welche sich als Prodrug eignen und deren Affinität zu CD22 nicht bestimmt wurde, da das aktive Molekül erst in vivo durch enzymatische Spaltung freigesetzt wird.

**Tabelle V**

| Nr. | Struktur |
|---|---|
| 16 | 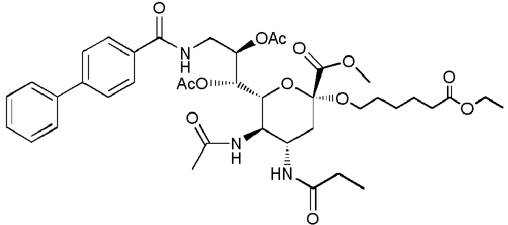 |
| 18 | 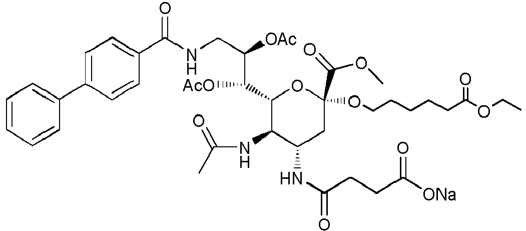 |
| 28 | 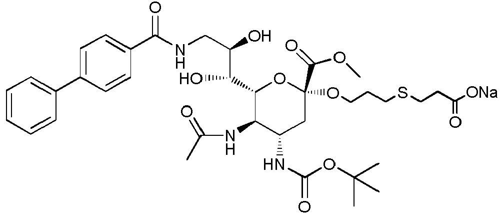 |
| 36 | 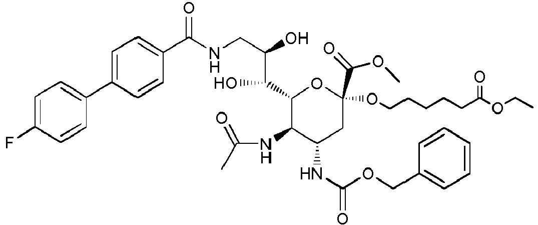 |
| 77 | 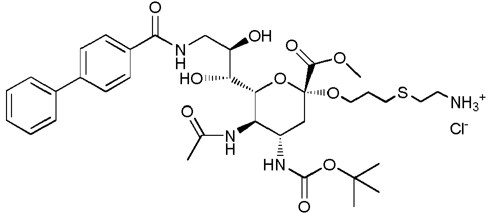 |

## Patentansprüche

1. Sialinsäurederivat der Formel (I), 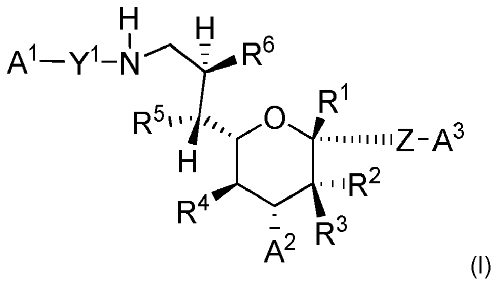 wobei die Symbole folgende Bedeutungen haben:
A¹ ist eine Gruppe D¹-[Y²-D²-]ₘ-;
D¹ ist ein mono- oder polycyclischer aromatischer, partiell ungesättigter oder gesättigter C₃-C₁₄-Kohlenwasserstoffrest oder ein mono- oder polycyclischer aromatischer, partiell ungesättigter oder gesättigter drei- bis zwölfgliedriger heterocyclischer Rest, wobei die genannten Reste unsubstituiert oder ein- oder mehrfach durch eine Gruppe X substituiert sind;
D² ist ein mono- oder polycyclischer aromatischer, partiell ungesättigter oder gesättigter C₃-C₁₄-Kohlenwasserstoffrest oder ein mono- oder polycyclischer aromatischer, partiell ungesättigter oder gesättigter drei- bis achtgliedriger heterocyclischer Rest, wobei die genannten Reste unsubstituiert oder ein- oder mehrfach durch eine Gruppe X substituiert sind;
Y¹ ist ~C(O)-, ~S(O)₂-, ~NHC(O)-, ~(C₁-C₂-Alkyl)-, ~(C₁-C₂-Alkyl)-C(O)-, ~CH=CH-C(O)-, ~C≡C-C(O)-, ~(C₁-C₂-Alkyl)-S(O)₂-, ~OC(O)-, ~(C₁-C₂-Alkyl)-OC(O)- oder ~(C₁-C₂-Alkyl)-NHC(O)- wobei ~ die Bindung zur Gruppe A¹ bezeichnet;
Y² ist -O-, -C(O)-, -S(O)₂-, -CH₂- oder eine Bindung;
A² ist
a) eine Gruppe -OS(O)₂OL oder
b) eine Gruppe -N(R^{x})-W;
W ist
a) eine Gruppe ~SO₃L, ~SO₂CF₃ oder ~SO₂NR^{x}₂ oder
b) eine Gruppe D³-Y³-;
Y³ ist eine Bindung oder eine Gruppe ~O(CO)NHS(O)₂-, ~NHC(O)-, ~OC(O)-, ~CH₂OC(O)-, ~S(O)₂-, ~C(O)-, ~(C₁-C₂-Alkyl)-C(O)-, ~(C₁-C₂-Alkyl)-NHC(O)- oder ~(C₁-C₂-Alkyl)-S(O)₂-, wobei - die Bindung zur Gruppe D³ bezeichnet;
D³ ist
a) C₁-C₆-Alkyl, wobei gegebenenfalls eine oder mehrere nicht terminale CH₂-Gruppen durch O, N(R^{x}) und/oder C(O) ersetzt sind und wobei in den genannten Gruppen gegebenenfalls ein oder mehrere H-Atome durch eine Gruppe X ersetzt sind,
b) ein mono- oder polycyclischer aromatischer, partiell ungesättigter oder gesättigter C₃-C₁₄-Kohlenwasserstoffrest oder ein mono- oder polycyclischer aromatischer, partiell ungesättigter oder gesättigter dreibis achtgliedriger heterocyclischer Rest, wobei die genannten Reste unsubstituiert oder ein- oder mehrfach durch eine Gruppe X substituiert sind;
A³ ist
a) C₁-C₈-Alkyl, wobei gegebenenfalls
a. eine oder mehrere nicht terminale -CH₂-Gruppen durch S, O, N(R^{x}) und/oder C(O) ersetzt sind oder
b. eine -CH₂CH₂CH₂-Gruppe durch 1,2-Phenyldiyl, 1,3-Phenyldiyl oder 1,4-Phenyldiyl ersetzt ist und wobei in den genannten Gruppen gegebenenfalls ein oder mehrere H-Atome durch eine Gruppe X ersetzt sind.
b) ein mono- oder polycyclischer aromatischer, partiell ungesättigter oder gesättigter C₃-C₁₄-Kohlenwasserstoffrest oder ein mono- oder polycyclischer aromatischer, partiell ungesättigter oder gesättigter dreibis achtgliedriger heterocyclischer Rest, wobei die genannten Reste unsubstituiert oder ein- oder mehrfach durch eine Gruppe X substituiert sind;
X ist gleich oder verschieden Halogen, Cyano, Nitro, Hydroxy, Mercapto, Amino, Carboxyl, Carboxymethyl, Hydroxylamino, Azido, B(OH₂), SO, SO₃M, OSO₃M, SO₂NH₂, SO₂CF₃, PO₃M, OPO₃M, Cyanomethyl, Alkyl, Halogenalkyl, Alkyloxy, Halogenalkyloxy, Alkylamino, Dialkylamino, Trialkylamino, Formyl, Alkylcarbonyl, Alkylsulfonyl, Alkylsulfoxyl, Alkylaminosulfonyl, Dialkylaminosulfonyl, Alkyloxycarbonyl, Alkylcarbonyloxy, Aminocarbonyl, Alkylaminocarbonyl, Alkylcarbonylamino, Dialkyl-aminocarbonyl, Oxo (=O), Thioxo (=S), C₁-C₈-Alkylimino (=N-C₁-C₈-Alkyl) oder C₁-C₈-Alkyloximino (=N-O-C₁-C₈-Alkyl), wobei die Alkylgruppen in diesen Resten 1 bis 6 Kohlenstoffatome enthalten;
m ist 0, 1 oder 2
Z ist ~O-, -S-, -ON=CH~, ~ON(R^{x})-, ~N(R^{x})- oder ~4-1H-(1,2,3)Triazol-1-yl-wobei - die Bindung zur Gruppe A³ bezeichnet;
R¹ ist C(O)OM;
R² ist H, F, Cl, NR^{x} oder OR^{x};
R³ ist H, F, Cl, NR^{x} oder OR^{x};
R⁴ ist N(R^{x})C(O)CH₂OH oder N(R^{x})C(O)R^{x};
R⁵, R⁶ sind gleich oder verschieden OH oder OR^{x};
L ist ein Kation;
M ist C₁-C₄-Alkyl oder ein Kation;
R^{x} ist gleich oder verschieden H, R^{y} oder R^{z};
R^{y} ist gleich oder verschieden C₁-C₄-Alkyl, Phenyl oder Benzyl und
R^{z} ist gleich oder verschieden -C(O)-C₁-C₄-Alkyl, -C(O)-Phenyl oder C(O)-CH₂-Phenyl,
wobei Alkylgruppen jeweils geradkettig, verzweigt oder cyclisch sind,
ein pharmakologisch verträgliches Salz oder Prodrug davon.

2. Sialinsäurederivat der Formel (I) gemäß Anspruch 1, wobei die Symbole in der Formel (I) folgende Bedeutungen haben:
A¹ ist eine Gruppe D¹-[Y²-D²-]ₘ-;
D¹ ist ein mono- oder polycyclischer aromatischer oder gesättigter C₃-C₁₄-Kohlenwasserstoffrest oder ein monocyclischer aromatischer, partiell ungesättigter oder gesättigter vier- bis sechsgliedriger heterocyclischer Rest, wobei die genannten Reste unsubstituiert oder ein- oder mehrfach durch eine Gruppe X substituiert sind;
D² ist eine Gruppe Phenylen-1,4-diyl, Phenylen-1,3-diyl, Pyridin-2,5-diyl, Pyridazin-3,6-diyl, Pyrimidin-2,5-diyl, Pyrimidin-2,6-diyl, Pyrazin-2,5-diyl, trans-Cyclobutan-1,3-diyl, trans-Cyclopentan-1,3-diyl, trans-Cyclohexan-1,4-diyl, Cuban-1,4-diyl, Thiophen-2,5-diyl, Pyrrol-2,4-diyl, Pyrrol-2,5-diyl, Pyrazol-1,3-diyl, Oxazol-2,4-diyl, Oxazol-2,5-diyl, 1,3,4-Oxadiazol-2,5-diyl, 1,2,4-Oxadiazol-3,5-diyl, Isooxazol-3,5-diyl, Imidazol-2,4-diyl, 2*H*-Tetrazol-2,5-diyl, 1*H*(1,2,4)-Triazol-2,5-diyl, 1*H*(1,2,3)-Triazol-1,4-diyl und 1*H*(1,2,3)-Triazol-1,5-diyl wobei die genannten Reste unsubstituiert oder ein- oder mehrfach durch eine Gruppe X substituiert sind;
Y¹ ist ~C(O)-, ~S(O)₂-, ~NHC(O)-, ~CH₂C(O)-, ~CH₂-S(O)₂-, ~OC(O)-,~CH₂-OC(O)- oder ~CH₂NHC(O)- wobei ~ die Bindung zur Gruppe A¹ bezeichnet;
Y² ist -O-, -CH₂- oder eine Bindung;
A² ist
a) eine Gruppe -OS(O)₂OL oder
b) eine Gruppe -N(R^{x})-W;
W ist
a) eine Gruppe ~SO₃L, ~SO₂CF₃ oder ~SO₂NR^{x}₂ oder
b) eine Gruppe D³-Y³-;
Y³ ist eine Bindung oder eine Gruppe ~NHC(O)-, ~OC(O)-,~CH₂OC(O)-, ~S(O)₂-, ~C(O)-, ~CH₂-C(O)-, ~CH₂-NHC(O)- oder ~CH₂-S(O)₂-, wobei - die Bindung zur Gruppe D³ bezeichnet;
D³ ist
a) ein C₁-C₆-Alkyl, wobei gegebenenfalls eine oder mehrere nicht terminale CH₂-Gruppen durch O ersetzt sind und wobei in den genannten Gruppen gegebenenfalls ein oder mehrere H-Atome durch eine Gruppe X ersetzt sind,
b) ein monocyclischer oder polycyclischer aromatischer oder gesättigter C₃-C₁₄-Kohlenwasserstoffrest oder ein monocyclischer aromatischer, partiell ungesättigter oder gesättigter vier- bis sechsgliedriger heterocyclischer Rest, wobei die genannten Reste unsubstituiert oder ein- oder mehrfach durch eine Gruppe X substituiert sind;
A³ ist ein C₃-C₈-Alkyl, wobei gegebenenfalls
a) eine oder mehrere nicht terminale -CH₂-Gruppen durch S oder O ersetzt sind oder
b) eine -CH₂CH₂CH₂-Gruppe durch 1,2-Phenyldiyl, 1,3-Phenyldiyl oder 1,4-Phenyldiyl ersetzt ist und wobei in den genannten Gruppen gegebenenfalls ein oder mehrere H-Atome durch eine Gruppe X ersetzt sind;
X ist gleich oder verschieden Halogen, Hydroxy, Amino, Carboxyl, Carboxymethyl, SO₃M, OSO₃M, SO₂NH₂, SO₂CF₃, Alkyl, Halogenalkyl, Alkyloxy, Alkylamino, Dialkylamino, Trialkylamino, Alkylsulfonyl, Alkyloxy-carbonyl, Alkylcarbonyloxy, Alkylaminocarbonyl, Alkylcarbonylamino oder Oxo (=O), wobei die Alkylgruppen in diesen Resten 1 bis 3 Kohlen-stoffatome enthalten;
m ist 0 oder 1;
Z ist ~O-, ~S- oder ~4-1*H*-(1,2,3)Triazol-1-yl- wobei ~ die Bindung zur Gruppe A³ bezeichnet;
R¹ ist C(O)OM;
R² ist H oder F;
R³ ist H, F, Cl oder OR^{x};
R⁴ ist NHC(O)CH₂OH oder NHC(O)CH₃;
R⁵, R⁶ sind gleich oder verschieden OH oder OR^{z};
L ist ein Kation;
M ist ein C₁-C₃-Alkyl oder ein Kation;
R^{x} ist gleich oder verschieden H, R^{y} oder R^{z};
R^{y} ist gleich oder verschieden C₁-C₃-Alkyl, Phenyl oder Benzyl und
R^{z} ist gleich oder verschieden -C(O)-C₁-C₃-Alkyl oder -C(O)-Phenyl.
ein pharmakologisch verträgliches Salz oder Prodrug davon.

3. Sialinsäurederivat der Formel (I) gemäß Anspruch 1 oder 2, wobei die Symbole in der Formel (I) folgende Bedeutungen haben:
A¹ ist eine Gruppe D¹-[Y²-D²-]ₘ-.
D¹ ist bevorzugt eine Gruppe Phenyl, Pyrimidin-5-yl, Napth-1-yl, Napth-2-yl, Thien-2-yl wobei die genannten Reste unsubstituiert oder ein- oder mehrfach durch eine Gruppe X substituiert sind;
D² ist eine Gruppe Phenylen-1,4-diyl, Pyridin-2,5-diyl, Pyrimidin-2,5-diyl oder Thiophen-2,5-diyl wobei die genannten Reste unsubstituiert oder ein- oder mehrfach durch eine Gruppe X substituiert sind;
Y¹ ist ~C(O)- oder ~CH₂C(O)- wobei ~ die Bindung zur Gruppe A¹ bezeichnet;
Y² ist eine Bindung;
A² ist
a) eine Gruppe -OS(O)₂OL oder
b) eine Gruppe -NH-W;
W ist
a) eine Gruppe ~SO₃L oder
b) eine Gruppe D³-Y³-;
Y³ ist eine Bindung oder eine Gruppe ~CH₂OC(O)-, ~OC(O)-, ~S(O)₂-, ~C(O)-oder ~CH₂-C(O)- wobei ~ die Bindung zur Gruppe D³ bezeichnet;
D³ ist eine Gruppe Methyl, Ethyl, Prop-2-yl, Pent-5-yl, Cyclopropyl, 1,1-Dimethylethyl, Phenyl, Thien-2-yl, Furan-2-yl, Imidazolidin-5-yl, Pyrazin-5-yl, Naphthalin-1-yl;
A³ ist eine Gruppe Pentan-1-yl oder Hexan-1-yl;
X ist gleich oder verschieden Fluor, Chlor, Hydroxy, Carboxyl, Methyl, Trifluormethyl, Methoxy, Dimethylamino oder Oxo (=O);
m ist 0 oder 1;
Z ist ~O-;
R¹ ist C(O)OM;
R² ist H;
R³ ist H oder OH;
R⁴ ist NHC(O)CH₃;
R⁵, R⁶ sind gleich oder verschieden OH oder OC(O)CH₃;
L ist ein Kation;
M ist Methyl, Ethyl oder ein Kation,
ein pharmakologisch verträgliches Salz oder Prodrug davon.

4. Sialinsäurederivat der Formel (I) gemäß einem der Ansprüche 1 bis 3, **gekennzeichnet durch** eine der Formeln la bis Ich, wobei die Symbole die in der Formel (I) angegebenen Bedeutungen haben: 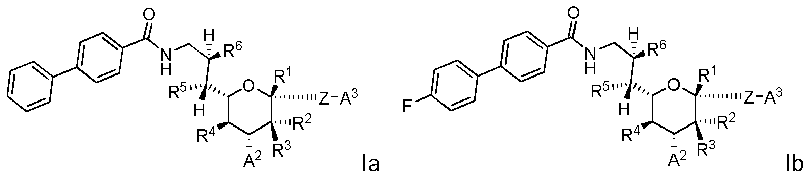 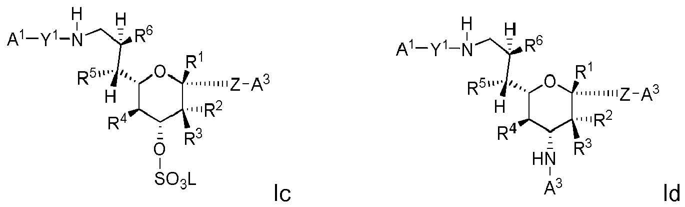 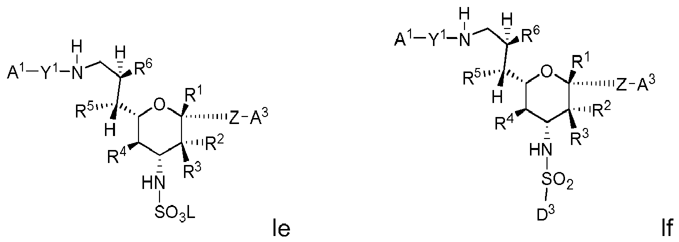 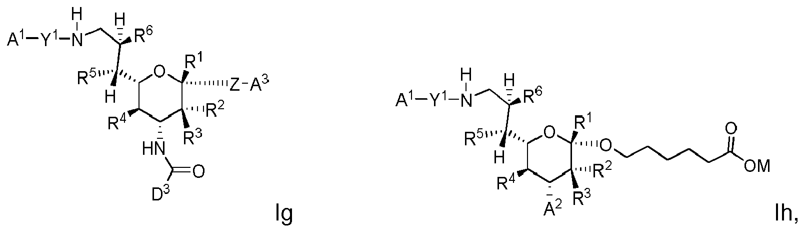 ein pharmakologisch verträgliches Salz oder Prodrug davon.

5. Sialinsäurederivat der Formel (I) gemäß einem der Ansprüche 1 bis 4, **gekennzeichnet durch** eine der Formeln (Iaa) - (lam), wobei die Symbole die in der Formel (I) angegebenen Bedeutungen haben: 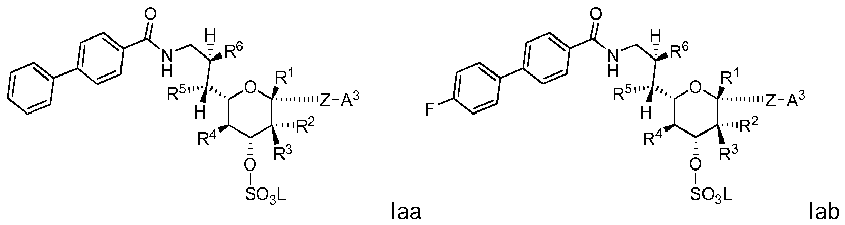 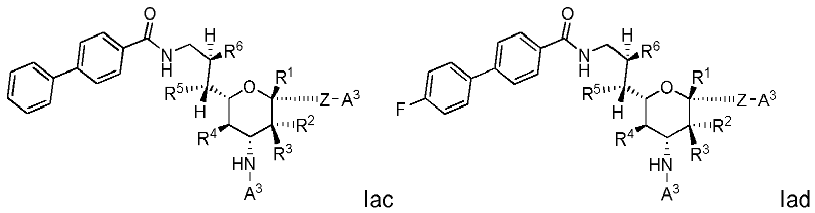 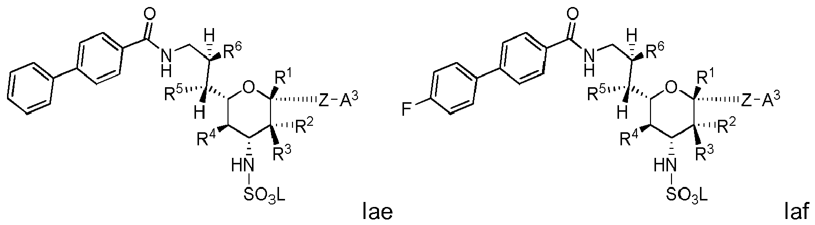 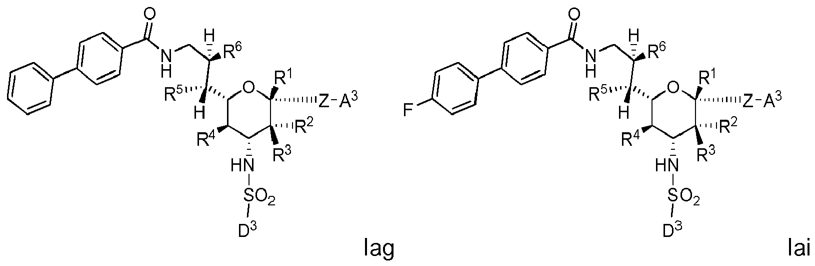 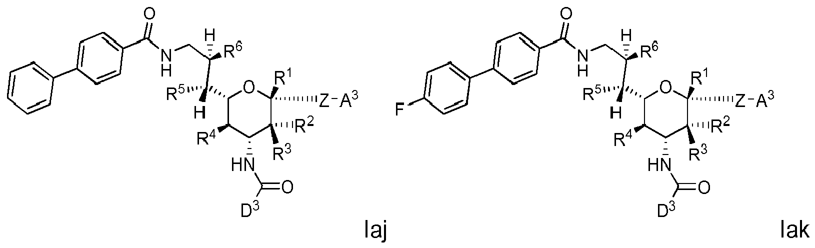 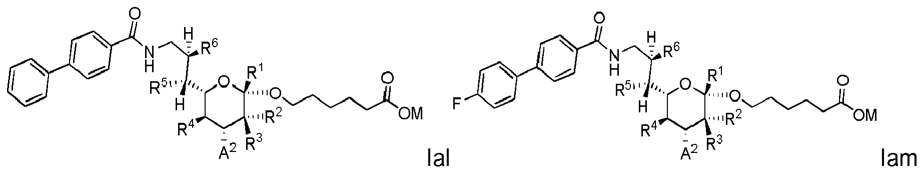 ein pharmakologisch verträgliches Salz oder Prodrug davon.

6. Sialinsäurederivat gemäß einem der Ansprüche 1 bis 5, **gekennzeichnet durch** eine der Formeln (Iba) - (Ibj), wobei die Symbole die in der Formel (I) angegebenen Bedeutungen haben: 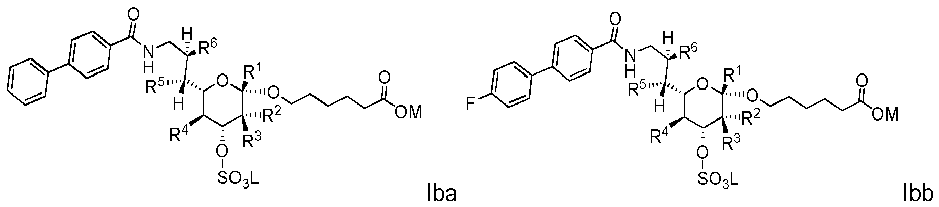 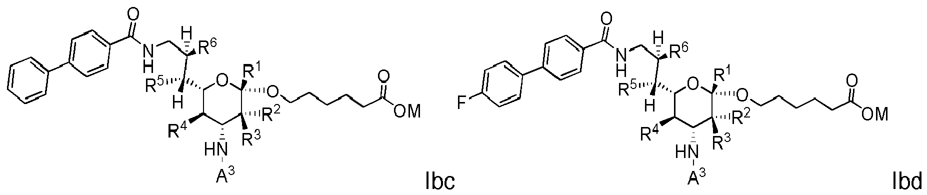 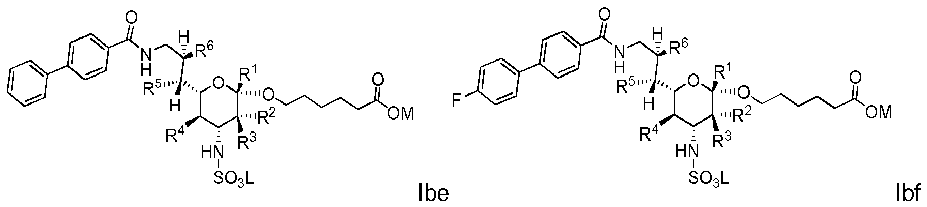 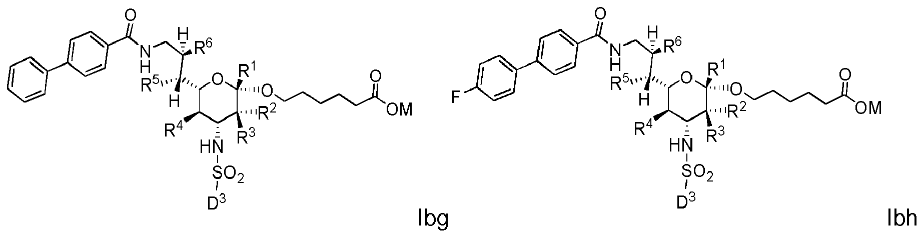 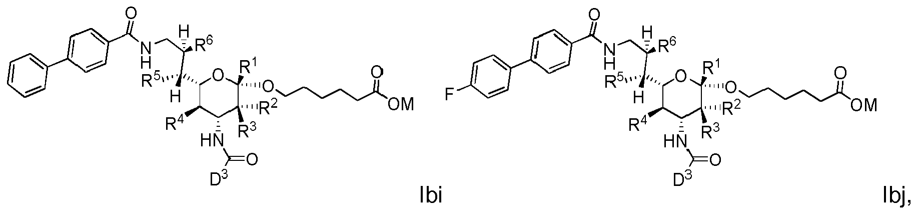 ein pharmakologisch verträgliches Salz oder Prodrug davon.

7. Sialinsäurederivat der Formel (I) nach einem der Ansprüche 1 bis 6, **gekennzeichnet durch** eine der Formeln (Ica) - (cj), wobei die Symbole die in der Formel (I) angegebenen Bedeutungen haben: 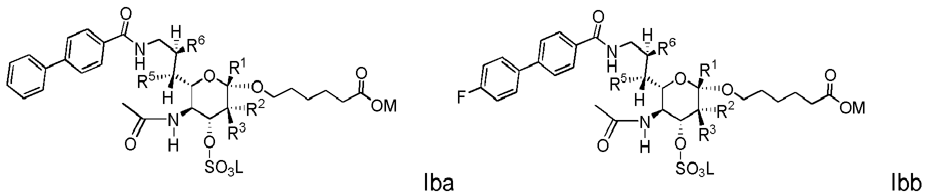 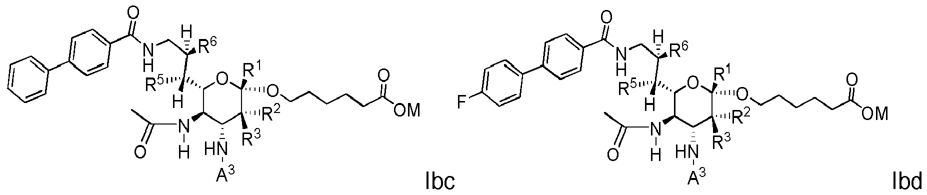 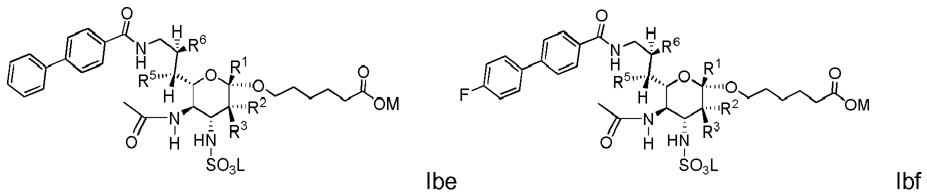 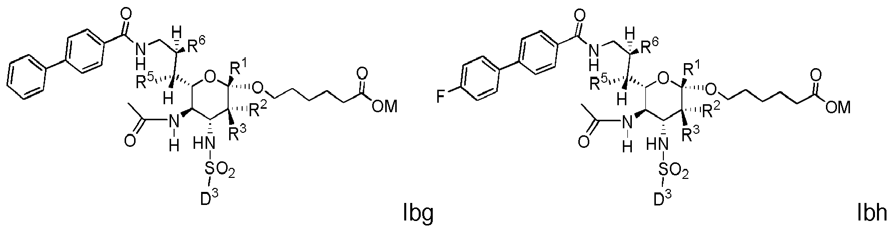 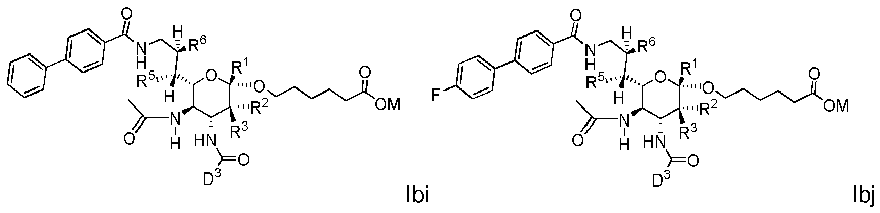 ein pharmakologisch verträgliches Salz oder Prodrug davon.

8. Pharmazeutische Zubereitung, enthaltend mindestens ein Sialinsäurederivat der Formel (I) oder ein pharmakologisch verträgliches Salz oder Prodrug davon gemäß einem der Ansprüche 1 bis 7 und einem pharmakologisch verträglichen Träger.

9. Sialinsäurederivat der Formel (I) oder ein pharmakologisch verträgliches Salz oder Prodrug davon gemäß einem der Ansprüche 1 bis 7 als Arzneimittel.

10. Sialinsäurederivat der Formel (I) oder ein pharmakologisch verträgliches Salz oder Prodrug davon gemäß einem der Ansprüche 1 bis 7 zur Behandlung oder Prävention von Allergien, Autoimmunerkrankungen, chronischen Entzündungen, Querschnittslähmung, multipler Sklerose, Krebs, Viruserkrankungen, bakteriellen Erkrankungen, parasitären Erkrankungen, Krankheiten, bei denen die Immunantwort im Rahmen der B-Zellenaktivierung gestört ist, bei Krankheiten der blutbildenden Organe und des Blutes, sowie zur Regulation des Immunsystems.

11. Sialinsäurederivat der Formel (I) oder ein pharmakologisch verträgliches Salz oder Prodrug davon gemäß einem der Ansprüche 1 bis 7 zur Verwendung bei der Herstellung eines Arzneimittels zur Regulation des Immunsystems, sowie zur Behandlung von Allergien, Autoimmunerkrankungen, chronischen Entzündungen, Querschnittslähmung, multipler Sklerose, Krebs, Viruserkrankungen, bakteriellen Erkrankungen, parasitären Erkrankungen, Krankheiten, bei denen die Immunantwort im Rahmen der B-Zellenaktivierung gestört ist, sowie bei Krankheiten der blutbildenden Organe und des Blutes.

12. Verfahren zur Regulation des Immunsystems sowie zur Behandlung von Krankheiten, deren Verlauf oder Aktivität durch die Siglec-Liganden beeinflusst werden kann, wobei man einer von der Krankheit betroffenen Person eine therapeutisch wirksame Menge eines Sialinsäurederivats der Formel (I) oder eines pharmakologisch verträglichen Salzes oder Prodrugs davon gemäß einem der Ansprüche 1 bis 7 verabreicht.

13. Verfahren zur Herstellung von Sialinsäurederivaten der Formel (I) gemäß einem der Ansprüche 1 bis 7 mit R³ = OH, wobei eine Verbindung der Formel (VI), 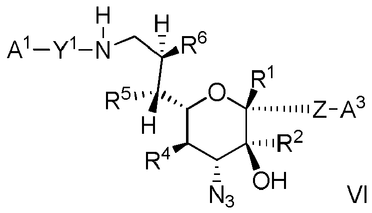 bei der die Symbole die gleichen Bedeutungen wie in der Formel (I) in einem der Ansprüche 1 bis 7 haben,
durch Reduktion des Azids, anschließende Umsetzung des entstandenen Amins mit einer Carbonsäure oder einem Sulfonylchlorid und gegebenenfalls Abspaltung von Schutzgruppen zu einem Sialinsäurederivat der Formel (I) mit R³ = OH umgesetzt wird.

14. Verfahren zur Herstellung von Sialinsäurederivaten der Formel (I) nach einem der Ansprüche 1 bis 7 mit R³ = H, wobei eine Verbindung der Formel (IV), 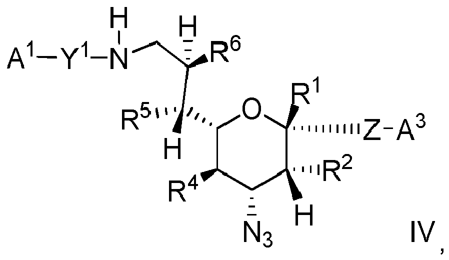 bei der die Symbole die gleichen Bedeutungen wie in der Formel (I) in einem der Ansprüche 1 bis 7 haben,
durch Reaktion des Azids, anschließende Umsetzung des entstandenen Amins mit einer aktivierten Carbonsäure, einem Sulfonylchlorid oder einem Sulfatierungsmittel und gegebenenfalls Abspaltung von Schutzgruppen zu einem Sialinsäurederivat der Formel (I) mit R³ = H umgesetzt wird.

15. Verfahren zur Herstellung von Sialinsäurederivaten der Formel (I) gemäß einem der Ansprüche 1 bis 7, wobei eine Verbindung der Formel (VIII) 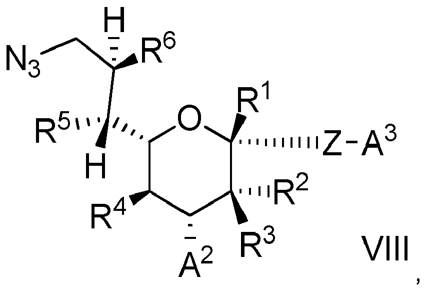 bei der die Symbole die gleichen Bedeutungen wie in der Formel (I) in einem der Ansprüche 1 bis 7 haben,
durch Reduktion des Azids, anschließende Umsetzung des entstandenen Amins mit einer aktivierten Carbonsäure, einem Sulfonylchlorid oder einem Sulfatierungsmittel und gegebenenfalls Abspaltung von Schutzgruppen zu einem Sialinsäurederivat der Formel (I) umgesetzt wird.

16. Zwischenprodukte zur Herstellung von Sialinsäurederivaten der Formel (I) nach einem der Ansprüche 1 bis 7, ausgewählt aus der Gruppe bestehend aus
a) Verbindungen der Formel (III), 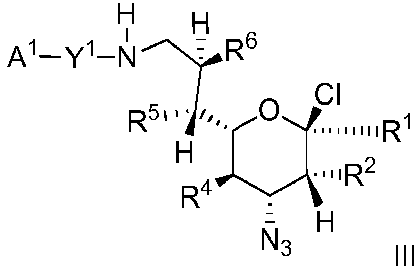
b) Verbindung der Formel (IV) 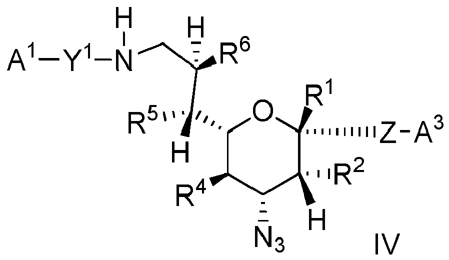 und
c) Verbindungen der Formel (VI), 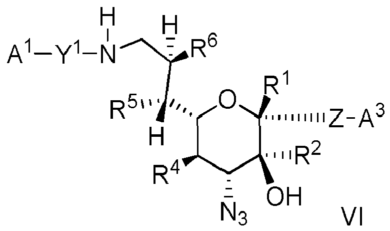 wobei die Symbole in den Formeln (III), (IV) und (VI) die gleichen Bedeutungen wie in Formel (I) in einem der Ansprüche 1 bis 7 haben.
